# EUROPEAN PATENT APPLICATION

(11) **EP 3 228 327 A2**
(43) Date of publication of application: **11.10.2017**
(21) Application number: 15859280.8
(22) Date of filing: 10.11.2015
(51) Int. Cl.: A61K 49/00, A61K 39/00

(54) **SUBSTANCE AND METHOD FOR USING THE SUBSTANCE MENTIONED FOR MODULATING THE ACTIVITY OF AN AGENT IN AN ORGANISM**

(30) Priority: 11.11.2014 RU 2014145275
(71) Applicant: Nikitin, Petr Ivanovich, Moscow 127562 (RU)
(72) Inventor: Nikitin, Petr Ivanovich, Moscow 127562 (RU)
(74) Representative: Jeck, Anton
(86) International application number: PCT/RU2015/000755
(87) International publication number: WO 2016/076760

(57) **Abstract**

The invention relates to biomedicine and nanomedicine, and makes it possible to enhance the diagnostic or therapeutic efficiency of an agent administered to the organism. The invention can be used to enhance methods of diagnostics and therapy of various diseases due to a more effective (passive or active) delivery of the agent to cell-targets, improved pharmacokinetic parameters of the agent (circulation time), etc. The essence of the invention consists in a substance for enhancing the diagnostic or therapeutic efficiency of an agent administered to the organism, this substance comprising a component that, upon administration of the substance to the organism, enables elimination from the bloodstream by the reticuloendothelial system of at least the objects, which circulate in the bloodstream but do not represent artificially created nanoparticles or microparticles, or opsonins that non-specifically bind to said component, and said elimination of said objects causes blockade, at least partial, of the reticuloendothelial system. The technical result of application of the invention consists in creation of the method for blockade of the cells of reticuloendothelial system by means of administration of a small amount of a foreign substance or a safe biocompatible substance, and also the result consists in enhancement (boosting) of the blockade effect.

## Description

The invention relates to the field of biomedicine and nanomedicine. The invention makes it possible to enhance the diagnostic or therapeutic efficiency of various agents (including that of diagnostic or therapeutic agents based on nano- and microparticles), to improve the efficiency of delivery of the agents to their targets in the organism (including directed or passive delivery of payload to cell-targets), to prolong blood circulation of the agents (circulation in blood flow (bloodstream)), which, inter alia, can be used to enhance the effectiveness of existing medicinal drugs, as well as for creation of new agents, preparations and methods for diagnostics, monitoring and therapy of various diseases and organism's states (conditions).

Lately, there has been a permanent growth observed in the use of nanotechnology in biology and medicine, as well as in other fields of science and technology. In treatment of diseases, nanoparticles offer a number of advantages over conventional molecular drugs, for example, they can transport large amounts of drugs protecting them from degradation; can carry out target drug delivery using even low-affinity receptors due to multi-point binding of the nanoparticles with the targets.

Besides, the nanoparticles are used in diagnostics of diseases, as labels in different biosensors and as contrasting agents. Application of various nanoparticles for treatment and diagnosis of such diseases as cancer, stroke, atherosclerosis, infectious diseases, etc. is of particular interest (McAteer, M.A., et al. In vivo magnetic resonance imaging of acute brain inflammation using microparticles of iron oxide, Nat Med. 13, 1253-8, 2007). For example, magnetic nanoparticles based on iron oxides are very interesting agents for diagnosis and therapy of diseases. At present, they are approved for intravenous injections to humans as contrasting substances in magnetic resonance imaging, undergo clinical trials for hyperthermia of cancer tumors and magnetically controlled purification of blood from toxins, cell separation; in addition, the magnetic particles are promising for magnetically controlled drug delivery.

The important task is to develop universal approaches based on such agents to increase the efficiency of drug delivery to targets in the organism and improve the effectiveness of diagnostics and therapy of various diseases and the organism's conditions (states).

A method is known (WO2009065065A1), in which an agent, which represents a nanoparticle, (liposome) is covered by polyethylene glycol (PEG) molecules to make it more hydrophilic and less "noticeable" for the cells of reticuloendothelial system (RES, sometimes referred to as the mononuclear phagocyte system). The RES cells are extremely efficient in removing nanoparticles from the bloodstream, reducing the blood circulation time of many particles to several minutes, thus compromising the theranostic ability of the particles. For example, in terms of directed drug delivery, the major portion of the particles is removed by the RES before they can even once encounter their target. The PEGylation decreases non-specific interactions of the agent and increases the time of its circulation in the bloodstream (blood circulation) [Kojima et al., Dendrimer-based MRI contrast agents: the effects of PEGylation on relaxivity and pharmacokinetics. Nanomedicine. 2011]; the efficiency of delivery of the agent to target also improves, as well as the diagnostic and/or therapeutic effects produced by the agent.

The drawbacks of this known method are as follows:
1) This approach may be inappropriate for complex particles, whose primary function is impaired by PEGylation. For some agents, surface properties are vital for operation. Accordingly, although PEGylation prolongs blood circulation of the agent, its productive activity may be compromised. This can be realized due to, for example, steric hindrance complications caused by PEG molecules that could affect receptor molecules, which have to bind the target, or the particles, whose behavior is determined by variability in the outer coating composition [see M.P. Nikitin, V.O. Shipuno-va, S.M. Deyev, P.I. Nikitin. Biocomputing based on particle disassembly, Nat Nano-technol, 9(9), 716-722 (2014)], or for the particles, whose activity consists in catalytic properties of their surface, etc.
2) The effect of PEGylation of many particles can be rather modest; PEGylation of some other particles not only leaves their characteristics unimproved, but also can impair them.
3) It is known that repeated injection of the PEGylated particles into the organism significantly decrease the effect of prolonged circulation of particles [Zhao et al. Repeated injection of PEGylated solid lipid nanoparticles induces accelerated blood clearance in mice and beagles. Int J Nanomedicine. Vol.7, 2891-2900, 2012], and sometimes induces accelerated blood clearance of the particles compared to the non-PEGylated ones.

A method is known (WO2014039874 A2) that is most similar to the proposed method, wherein passive delivery of particles into monocytes ("passive" in the sense that it is not mediated by any specific receptors) is improved if a fat emulsion (in particular, Intralipid® pretreatment) is administered into bloodstream besides (along with) the agent. The administration of Intralipid® saturates the cells of the reticuloendothelial system (in this case, mainly Kupffer cells of liver) and cause their blocking (blockade) in terms of their phagocytic activity. Because of this, the agent (magnetic nano- and microparticles) administered after the Intralipid® pretreatment circulates in the bloodstream longer and is more efficiently ingested (cleared) by the circulating in blood monocytes/macrophages than in the case without Intralipid adminitration. This way, the agent delivery into monocytes is enhanced, and that can be used for diagnostics and therapy of certain diseases.

The drawbacks of this known method are as follows:
1) Extremely high dose of the fat emulsion of 2g/kg is required for the RES blocking. Such high dose of a foreign substance may have a significant unwanted and negative effect on the organism.
2) Even this extremely high dose of the fat emulsion produces only slight effect of the RES blockade in terms of prolongation of circulation time, which is 3.1-fold for nanoparticles and 2.5-fold for microparticles.
3) This method provides the agent delivery solely to actively phagocytizing blood cells, i.e., to monocytes and macrophages, and the delivery is realized not through the specific activity of the agents but rather through the activity of those cells, whose mission in the organism, among others, is scavenging of foreign particles. To elucidate further, the method does not provide targeting (targeted drug delivery) of certain cells due to specific activity of the agent, for example, by means of the agent-conjugated receptors (e.g., antibodies) specific to the surface markers of cell-targets. Instead, the method produces changes in biodistribution of the particles over the organs and cells, which intrinsically phagocytize foreign particles in the organism.
4) In this method, administration of the pretreatment, which blocks the RES, considerably changes blood composition. For example, the fats (as pretreatment components), while present in high concentrations in blood, can interact with potential targets of the agent. This interaction can have undesirable effect on the target or impede interaction of the agent with the targets, e.g., by hiding it (if the target is a surface cell marker), inhibit phagocytosis of the target cells, and forbid the agent internalization into the target cells, etc. This, in turn, may considerably reduce the agent efficiency in spite of prolongation of its circulation in the bloodstream.

Thus, the required technical result consists in development of a method for enhancing the diagnostic or therapeutic efficiency of agents, improving the efficiency of the agent's delivery and/or prolongation circulation of the agents (including the agents based on nanoparticles or microparticles) in the bloodstream of the organism (preferably applicable for enhancing the effectiveness of the widest possible range of agents) through administration into the organism of a minimal amount of potentially foreign and/or toxic objects that preferably minimally change the blood composition.

To achieve this technical result, a substance (compound, formulation, composition) is proposed for enhancing the diagnostic or therapeutic efficiency of the agent administered to the organism (subject), this substance comprising a component that, upon administration of the substance to the organism, stimulates (induces) elimination (clearance) from the blood (bloodstream) by the reticuloendothelial system of at least the objects, which circulate in the bloodstream but do not represent artificially created nanoparticles or microparticles, or opsonins that non-specifically bind to said component, wherein said elimination of said objects causes blocking, at least partial, of the reticuloendothelial system.

It is implied that the blocking is realized not via RES saturation solely with said component; the component merely stimulates elimination of said objects. Preferably, the component is a separate formulation (matter, preparation, dosage form) that can interact with said objects or preferably bind to said objects, or react with said objects chemically or biochemically, or, preferably, enzymatically.

In this case, the stimulation of the said elimination (clearance) by said component means that upon administration of said substance, which does not comprise said component, elimination (clearance) of said objects would be performed slower, preferably, much (several times) more slowly, or, even more preferably, the RES blockade would not be realized.

Under the blockade of reticuloendothelial system, a condition is understood, in which the ability of the reticuloendothelial system to eliminate entities from the bloodstream is reduced, in particular, this may occur as a result of active phagocytosis of some objects by the cells of the RES. At that, in particular, a decrease in the phagocytosis activity of these RES cells and, as a result, an increase in blood circulation of the administered agents can be observed. Herein, a substantial (!) increase in blood circulation of said agent is preferable. However, such increase in circulation of the agent can be absent if, for example, the agent can quickly identify its target in the organism and bind to it. Then the RES blockade will allow the agents to avoid uptake by the RES cells and to bind to their target but because of the quick binding to the target, the agent will be cleared from the bloodstream almost as quickly. However, the changed biodistribution of the agents between the RES cells and target will be achieved.

Besides, a substance for delivery of the agent into the organism (subject), said substance comprising a component that, upon administration of said substance to the organism, stimulates elimination from the bloodstream by the reticuloendothelial system of at least the objects, which circulate in the bloodstream but do not represent artificially created nanoparticles or microparticles, or opsonins that non-specifically bind to said component, wherein said elimination of said objects causes blocking, at least partial, of the reticuloendothelial system.

Besides, a substance characterized in that said objects are cells.

Besides, a substance characterized in that said objects are erythrocytes.

Besides, a substance characterized in that said objects are thrombocytes.

Besides, a substance characterized in that said objects are leucocytes.

Besides, a substance characterized in that said objects are molecules.

At this, as mentioned above, it is important that these molecules do not act solely as opsonins, there is additional interaction between the component and said objects because it is clear that if the component is, for example, an artificial particle, then its elimination will be almost always accompanied by elimination from the bloodstream of opsonins (various molecules such as immunoglobulins, proteins of the complement system, etc. that non-specifically bind to said particles and mediate their phagocytosis by the RES cells).

Besides, a substance characterized in that said objects are a part of said substance but are not comprised in said component.

Besides, a substance characterized in that said objects are cells or molecules of the organism.

Besides, a substance characterized in that said objects are eliminated from free circulation in the bloodstream.

Under the free circulation in the bloodstream, the circulation in free form is understood, for example, if erythrocyte is absorbed by a macrophage circulating in the bloodstream then the erythrocyte is considered to be eliminated from the free circulation in the bloodstream.

Besides, a substance characterized in that said objects are cells that have not left the organism.

Besides, a substance characterized in that said objects are cells or molecules that got to the organism artificially.

Besides, a substance characterized in that said objects are cells or molecules that has got to the organism by transfusion of blood or donor blood constituents (components).

Besides, a substance characterized in that said objects are cells or molecules, which are comprised in said substance but are different from said component.

Besides, a substance characterized in that said component specifically interacts with said objects, and said interaction causes said their elimination by the reticuloendothelial system.

Besides, a substance characterized in that said interaction of said component with said objects is binding of said component with said objects.

Besides, a substance characterized in that said component non-covalently interacts with said objects.

Besides, a substance characterized in that said component forms a specific complex with said objects by means of direct or indirect recognition and binding to said objects.

Besides, a substance characterized in that said component comprises at least antibody that forms said specific complex with said objects by means of direct or indirect recognition and binding to said objects.

Besides, a substance characterized in that said direct or indirect recognition and binding of said component to said objects causes mainly phagocytosis of said objects by the organism's cells mediated by the Fc-receptor.

Besides, a substance characterized in that said antibody is targeted against erythrocytes or thrombocytes of the organism or their allogeneic variants.

Besides, a substance characterized in that said component comprises at least an antibody (or its analog), which is highly homologous to the antibodies of the organism, including autologous, allogeneic, humanized or chimeric antibody.

Besides, a substance characterized in that said component comprises at least a monoclonal antibody.

Besides, a substance characterized in that said component stimulates elimination of said objects, which are cells comprised in said substance, by means of recognition of said cells and facilitating of their phagocytosis by the reticuloendothelial system.

Besides, a substance characterized in that said component comprises at least several antibodies targeted at several types of cells or molecules of the organism.

Besides, a substance characterized in that said component comprises at least several antibodies, which are at least monoclonal or at least highly homologous to the antibodies of the organism (or their combinations and variants), including autologous, allogeneic, humanized or chimeric antibodies against cells or molecules of the organism, or their allogeneic analogs.

Besides, a substance characterized in that said component comprises at least an antibody linked [bound] with other substances that comprise, among other things, molecules, particles, cells or their combinations.

Besides, a substance characterized in that said antibody is targeted against erythrocytes of the organism.

Besides, a substance characterized in that said antibody is targeted against thrombocytes of the organism.

Besides, a substance characterized in that said antibody is targeted against leucocytes of the organism.

Besides, a substance characterized in that said direct or indirect recognition and binding of said component to said objects causes phagocytosis of said objects by other cells of the organism.

Besides, a substance characterized in that said elimination of said objects by the reticuloendothelial system is due to their agglutination induced by said component of said substance.

Besides, a substance characterized in that said elimination of said objects by the reticuloendothelial system is due to their ageing induced by said component of said substance.

Besides, a substance characterized in that said elimination of said objects by the reticuloendothelial system is due to their damage induced by said component of said substance.

Besides, a substance characterized in that said elimination of said objects by the reticuloendothelial system is due to their modification induced by said component of said substance.

Besides, a substance characterized in that said component causes increased phagocytosis activity of the cells of the organism's reticuloendothelial system, which eliminates said objects from the bloodstream.

Besides, a substance characterized in that said component is a chemical compound, or a molecule, or a particle, or a cell (including a bacterium, a leucocyte, etc.) or a combination of these entities, but said component does not comprise erythrocytes.

Besides, a substance characterized in that said component is a chemical compound, or a molecule, or a particle, or a cell (including a bacterium, a leucocyte, etc.) or a combination of these entities, but said component does not comprise objects obtained by any modification of erythrocytes.

Besides, a substance characterized in that said component differs from an erythrocyte or a modified erythrocyte.

Besides, a substance, administration of which to the organism causes said blocking of the reticuloendothelial system so that time of the agent circulation in the bloodstream increases.

Besides, a substance, administration of which to the organism causes said blocking of the reticuloendothelial system so that the half-life of the agent's circulation in bloodstream increases not less than by 1.2 times, more preferably by 1.5 times, more preferably by 1.75 times, more preferably by 2 times, more preferably by 3 times, more preferably by 5 times, more preferably by 7 times, more preferably by 10 times, more preferably by 15 times, more preferably by 20 times and even more preferably by 25 times as compared with the half-life of the agent without administration of the substance.

Besides, a substance, administration of which to the organism causes said blocking of the reticuloendothelial system so that the half-life of the agent's circulation in bloodstream increases not less than by 5 times (5-fold) as compared with the half-life of the agent without administration of the substance.

Besides, a substance, for which said blockade of the reticuloendothelial system is temporary.

Besides, a substance characterized in that the mass of the component does not exceed 20% of the mass of the objects that cause blockade of the reticuloendothelial system.

Besides, a substance characterized in that the mass of the component does not exceed 75%, more preferably 50%, more preferably 25%, more preferably 10%, more preferably 1%, more preferably 0.1 %, more preferably 0.01% and even more preferably 0.001% of the mass of the objects eliminated by the reticuloendothelial system from the bloodstream to provide the RES blockade. Under the mass of cells, for example, one can understand the mass with the water contained in them and without it.

Besides, a substance characterized in that the dose of the active components, presence of which induces blockade of the reticuloendothelial system, does not exceed 5 mg/kg of the organism weight.

Besides, a substance characterized in that the dose of the active components, presence of which induces blockade of the reticuloendothelial system, does not exceed at least one of the following doses: 5, 2.5, 1, 0.5, 0.25, 0.1, 0.05, 0.025, 0.01, 0.005, 0.0025, 0.0015 g/kg of the organism weight.

Besides, a substance, administration of which to the organism increases the efficiency of passive or active delivery of said agent to a target by at least 2 times, more preferably by 3 times, more preferably by 5 times, more preferably by 7 times, and even more preferably by 10 times as compared with the delivery of the agent to the target without administration of the substance.

Besides, a substance characterized in that said component comprises at least monoclonal antibody or, at least antibody (or its analog) that is highly homologous to the antibodies of the organism, including autologous, allogeneic, humanized or chimeric antibody, which can specifically directly or indirectly recognize and bind to the cells or molecules that circulate in the bloodstream (naturally circulating or brought from outside).

Besides, a substance characterized in that said antibody (antibodies) is linked with other objects, which comprise, inter alia, molecules, particles, cells or their combinations.

Besides, a substance characterized in that administration of said antibody causes agglutination of cells or molecules.

Besides, a substance characterized in that said direct or indirect recognition and binding of said antibody to cells causes phagocytosis of said cells.

Besides, a substance characterized in that said direct or indirect recognition and binding of said antibody to cells induces mainly Fc-mediated phagocytosis of said cells.

Besides, a substance, administration of which to the organism increases the diagnostic or therapeutic efficiency of said agent by at least 1.3 times as compared to delivery of the agent without the said substance administration.

Besides, a substance, for which said agent comprises, inter alia, at least one of the following nanoparticle or microparticle: magnetic, fluorescent, protein (including cross-linked, polymerized or aggregated protein), polymer (including polystyrene, dextran, polypeptide, glycolic acid polylactide or other polymers and block-copolymer, etc.) or crystalline (gold, silver, semiconductor, etc.) nanoparticle or microparticle.

Besides, a substance, for which said agent represents nanoparticles or microparticles (including the particles comprising nanoparticles or microparticles).

Besides, a substance characterized in that said agent represents a nanoparticle or microparticle, whose average size in one dimension (i.e. dispersion taken into account) exceeds 10 nm, or 19 nm, or 29 nm, or 29 nm, or 49 nm, or 74 nm, or 99 nm, or 199 nm, or 299 nm, or 399 nm, or 499 nm, or 599 nm, or 699 nm, or 799 nm, or 899 nm, or 999 nm, or 1299 nm, or 1499 nm, or 1999 nm, or 2499 nm, or 2999 nm.

Besides, a substance characterized in that said agent represents a nanoparticle or microparticle, whose average size in one dimension (i.e. dispersion taken into account) does not exceed 10 nm, or 19 nm, or 29 nm, or 29 nm, or 49 nm, or 74 nm, or 99 nm, or 199 nm, or 299 nm, or 399 nm, or 499 nm, or 599 nm, or 699 nm, or 799 nm, or 899 nm, or 999 nm, or 1299 nm, or 1499 nm, or 1999 nm, or 2499 nm, or 2999 nm.

Besides, a substance characterized in that said agent is used for diagnostics or therapy of diseases or the organism's conditions, which include one or several following diseases: cancer, atherosclerosis, stroke, myocardial infarction, including diagnostic of diseases or the organism's conditions (states) using angiocontrasting.

Besides, a substance characterized in that said agent implements an imaging function (performs imaging) by producing of a detectible signal, including at least one from the following list: fluorescent signal, luminescent signal, PET signal, MRI contrasting signal, ultrasound contrasting signal, X-ray contrasting signal, magnetic signal or a signal due to plasmon resonance, or other method based on absorption by the agent of light, or electromagnetic, or acoustic waves (signals), etc.

Besides, a substance characterized in that said agent implements agent performs therapeutic function, for example, by means of delivery to targets of the agents for hyperthermia (magnetic or gold particles), of cytostatic or cytotoxic compounds, or medicinal compounds, including low-molecular-weight, enzyme, radioactive, chemotherapeutic compounds for photodynamic therapy.

Besides, a substance for enhancement of diagnostic or therapeutic effect of an agent administered to the organism; said substance comprising at least a component, which, being administered to the organism, causes at least partial blockade of the reticuloendothelial system with cells.

Besides, a substance for delivery an agent to the organism; said substance comprising at least a component, which, being administered to the organism, causes at least partial blockade of the reticuloendothelial system with cells.

Here, under the blockade of the reticuloendothelial system with cells it is implied that the organs of the reticuloendothelial system eliminate a substantial amount of said cells, by which the RES is blocked; at this, it is clear that along with said cells, the RES eliminates other objects (because this is one of the major functions of the RES cells. Herewith, for example, in one of the embodiment of the invention, administration of said substance considerably increases the amount of elmininated cells (as compared with the case without the substance administration), and their elimination plays a significant role in the RES saturation. In the preferable embodiment of the invention, the amount of cells eliminated from the bloodstream during a certain period of time (10 minutes, 1 hour, 3 hours, 6 hours, 9 hours, 12 hours, 15 hours, 18 hours, 24 hours, 2 days, 3 days, 4 days, 5 days, 7 days or 10 days) substantially increases (by 1.5, 2, 3, 4, 5, 7, 10, 20, 30, 40, 50, 75 or more times) as compared with the amount of cells eliminated from the bloodstream by the RES cells without administration of said substance.

Besides, a substance characterized in that said component comprises at least antibody, which is highly homologous to the antibodies of the organism, including autologous, allogeneic, humanized or chimeric antibody, against said cells, which provide said blockade of the reticuloendothelial system.

Besides, a substance characterized in that said component induces (causes) increased elimination of the cells, which circulate in the bloodstream, by the reticuloendothelial system; due to this effect, said, at least partial, blockade with cells is achieved.

Besides, a substance characterized in that said component comprises said cells, with which the reticuloendothelial system is blocked at least partially due to their quick clearance (elimination) from the circulation in the bloodstream so that said clearance (elimination) is sufficient for said blockade of the reticuloendothelial system.

Besides, a substance characterized in that said cells, with which said blockade of the reticuloendothelial system is caused, are erythrocytes.

Besides, a substance characterized in that said cells, with which said blockade of the reticuloendothelial system is caused, are thrombocytes.

Besides, a substance characterized in that said cells, with which said blockade of the reticuloendothelial system is caused, are leucocytes.

Besides, a substance characterized in that said cells are cells administered to the organism by transfusion of the donor's or organism's material.

Besides, a substance characterized in that said cells are processed variants of the organism's cells or of allogeneic cells.

Besides, a substance characterized in that said processed variants of the organism's cells or of allogeneic cells are cells including cells incubated with specific antibody, that directly or indirectly recognize and bind with said variants of the organism's cells or allogeneic cells.

Besides, a substance characterized in that said specific antibody is an antibody (or its analog), which is highly homologous to the antibodies of the organism, including autologous, allogeneic, humanized or chimeric antibody.

Besides, a substance characterized in that said specific antibody is a monoclonal antibody.

Besides, a substance characterized in that said cells are comprised in said component.

Besides, a substance characterized in that said cells are the cells that have not left the organism.

Besides, a substance characterized in that said component specifically interacts with the cells circulating in the bloodstream, and said interaction induces (leads to) their clearance (elimination) from the bloodstream by the reticuloendothelial system, and said clearance (elimination) induces (causes) said blockade of the reticuloendothelial system.

Besides, a substance characterized in that said interaction of said component with the cells circulating in the bloodstream is binding of said component with said cells.

Besides, a substance characterized in that said component interacts with the cells circulating in the bloodstream noncovalently.

Besides, a substance characterized in that said component forms a specific complex with said cells by means of direct or indirect recognition and binding to said cells.

Besides, a substance characterized in that said component comprises at least an antibody, which forms said specific complex with said cells by means of direct or indirect recognition and binding to said cells.

Besides, a substance characterized in that said antibody is targeted against the organism's erythrocytes.

Besides, a substance characterized in that said antibody is targeted against the organism's thrombocytes.

Besides, a substance characterized in that said antibody is targeted against the organism's leucocytes.

Besides, a substance characterized in that said direct or indirect recognition and binding of said component to cells causes phagocytosis of said cells by other cells of the organism.

Besides, a substance characterized in that said direct or indirect recognition and binding of said component to said objects induces (causes) mainly phagocytosis of said objects by the organism's cells mediated by the Fc-receptor.

Besides, a substance characterized in that said cells are agglutinated cells, and either are comprised in said component or agglutinate when affected by said component.

Besides, a substance characterized in that said cells are damaged cells, and either are comprised in said component or are damaged when affected by said component.

Besides, a substance characterized in that said cells are modified cells, and either are comprised in said component or get modified when affected by said component.

Besides, a substance characterized in that said modified cells are enzymatically modified by means of, among other things, neuraminidase, trypsin, galactosidase, etc.

Besides, a substance characterized in that said modified cells are chemically modified by means of, among other things, glutaraldehyde, hydrogen peroxide.

Besides, a substance characterized in that said cells are aged, and either are comprised in said component or are aged when affected by said component.

Besides, a substance characterized in that said component comprises at least several antibodies, which target several different types of the organism's cells or their allogeneic variants.

Besides, a substance characterized in that said component comprises at least several at least monoclonal antibodies or antibodies, which are at least highly homologous to the antibodies of the organism (or their combinations and variants), including autologous, allogeneic, humanized or chimeric antibodies, against the organism's cells or their allogeneic variants.

Besides, a substance characterized in that said component differs from an erythrocyte or modified erythrocyte.

Besides, a substance characterized in that said agent is nanoparticles or microparticles (including the particles that comprise nanoparticles or microparticles).

Besides, a substance characterized in that said agent is a nanoparticle or microparticle, the size of which in one of the dimensions exceeds 10 nm.

Besides, a substance, administration of which to the organism induces (causes) said blockade of the reticuloendothelial system so that the half-life of the agent's circulation in bloodstream increases not less than by 1.2 times, more preferably by 1.5 times, more preferably by 1.75 times, more preferably by 2 times, more preferably by 3 times, more preferably by 5 times, more preferably by 7 times, more preferably by 10 times, more preferably by 15 times, more preferably by 20 times and even more preferably by 25 times as compared with the half-life of the agent without administration of the substance.

Besides, a substance characterized in that the dose of the active components, which induces (causes) blocking of the reticuloendothelial system, does not exceed 5 mg/kg of the organism weight.

Besides, a substance characterized in that the dose of the active components, which induces (causes) blocking of the reticuloendothelial system, does not exceed at least one of the following doses: 5, 2.5, 1, 0.5, 0.25, 0.1, 0.05, 0.025, 0.01, 0.005, 0.0025, 0.0015 g/kg of the organism weight.

Besides, a substance, administration of which to the organism increases the efficiency of passive or active delivery of said agent to a target by at least 2 times, more preferably by 3 times, more preferably by 5 times, more preferably by 7 times, and even more preferably by 10 times as compared with the delivery of the agent to the target without administration of the substance.

Besides, a substance, administration of which to the organism increases the diagnostic or therapeutic efficiency of said agent by at least 1.3 times as compared with the agent delivery to the target without administration of said sunstance.

Besides, a substance characterized in that said agent comprises, inter alia, at least one of the following nanoparticles or microparticles: magnetic, fluorescent, protein (including cross-linked, polymerized or aggregated protein), polymer (including polystyrene, dextran, polypeptide, glycolic acid polylactide or other polymers and block-copolymer, etc.) or crystalline (gold, silver, semiconductor, etc.) nanoparticle or microparticle.

Besides, a substance characterized in that said agent is used for diagnostics or therapy of diseases or the organism's conditions, which include one or several following diseases: cancer, atherosclerosis, stroke, myocardial infarction, including angiocontrasting-based procedures for diagnostics of diseases or the organism's conditions (states).

Besides, a substance characterized in that said agent implements visualization by producing a detectible signal, including at least one from the following list: fluorescent signal, luminescent signal, PET signal, MRI contrasting signal, ultrasound contrasting signal, X-ray contrasting signal, magnetic signal or a signal due to plasmon resonance.

Besides, a substance characterized in that said agent implements agent performs therapeutic function, for example, by means of delivery to targets of cytostatic or cytotoxic compounds, or medicinal compounds, including low-molecular-weight, enzyme, radioactive, chemotherapeutic compounds for photodynamic therapy, hyperthermia.

Besides, a substance administered to the organism for enhanacement of the diagnostic or therapeutic performance of said agent, said substance comprising at least an antibody (or its analog), which is highly homologous to the antibodies of the organism, including autologous, allogeneic, humanized or chimeric antibody, against erythrocytes or thrombocytes.

Besides, a substance that comprises, inter alia, erythrocytes and thrombocytes.

Besides, a substance characterized in that said antibody are bound with erythrocytes or thrombocytes of the organism or their allogeneic variants.

Besides, a substance, which is administered to the organism for enhanacement of the diagnostic or therapeutic performance of said agent or for delivery of the agent, or for prolongation of circulation of the agent in the bloodstream; said substance comprising at least an antibody (or its analog), which is highly homologous to the antibodies of the organism, including autologous, allogeneic, humanized or chimeric antibody, against erythrocytes or thrombocytes.

Besides, a substance for delivery of the agent, said substance comprising at least an antibody (or its analog), which is highly homologous to the antibodies of the organism, including autologous, allogeneic, humanized or chimeric antibody, against erythrocytes or thrombocytes.

Besides, a substance for prolongation of circulation of said agent in the bloodstream; said substance comprising at least an antibody (or its analog), which is highly homologous to the antibodies of the organism, including autologous, allogeneic, humanized or chimeric antibody, against erythrocytes or thrombocytes.

Besides, a method for diagnosis or therapy of diseases or the organism's conditions (states), wherein:
i) at least one of the abovementioned substances is administered to the organism;
ii) before or after administration of said substance, or simultaneously with administration of said substance (co-administered with said substance), an agent is administered, which mediates the diagnostic or therapy action.

Besides, a method characterized in that said agent is administered not less than 6 hours and not more than 18 hours after administration of said substance.

Besides, a method characterized in that said agent is administered not less than 11 hours and not more than 13 hours after administration of said substance.

Besides, a method characterized in that said agent is administered not less than 20 days, more preferably 15 days, more preferably 10 days, more preferably 7 days, more preferably 5 days, more preferably 4 days, more preferably 3 days, more preferably 2 days, more preferably 1 day, and even more preferably 15 hours after administration of said substance.

Besides, a method characterized in that said administration to the organism of said substance increases the efficiency of diagnostic or therapeutic action of said agent due to the prolonged circulation of said agent in the bloodstream.

Besides, a method characterized in that along with administration of said substance and said agent, a recovery preparation (recovery agent, recovery formulation, regenerating preparation) is administered to the organism; said recovery preparation promotes an increase (compared to the case without administration of said recovery preparation) of the number of cells or molecules, clearance (elimination) of which from the bloodstream is induced (stimulated) by said component of said substance.

Besides, a method characterized in that said recovery preparation comprises the organism's blood or the organism's blood components, or donated blood, or donated blood components.

Besides, a method characterized in that said recovery preparation comprises erythropoietin.

Besides, a substance for delivery of an agent to the organism and/or enhancing the diagnostic or therapeutic efficiency of the agent, this substance comprising at least a component or components, which: i) upon administration to the organism, stimulate increased elimination (clearance) from circulation in the bloodstream of cells or molecules of the organism, or ii) upon administration to the organism, stimulate increased elimination from circulation in the bloodstream of cells or molecules administered to the organism, or iii) are cells themselves, which, after administration of said substance to the organism, are quickly eliminated (cleared) from circulation in the bloodstream (blood circulation); in any of the mentioned cases i)-iii) said increased or quick elimination of said cells or molecules from circulation in the bloodstream induces (leads to) prolongation of circulation of said agent in the bloodstream.

Besides, a substance for delivery of an agent to the organism and/or facilitating delivery of the agent to the organism and/or enhancing the diagnostic or therapeutic efficiency of the agent, this substance comprising at least a component or components, which: i) upon administration to the organism, stimulate increased elimination (cleared) from circulation in the bloodstream of cells or molecules of the organism, or ii) upon administration to the organism, stimulate increased elimination from circulation in the bloodstream of cells or molecules administered to the organism, or iii) are cells themselves, which, after administration of said substance to the organism, are quickly eliminated from circulation in the bloodstream; in any of the mentioned cases i)-iii) said increased or quick elimination of said cells or molecules from circulation in the bloodstream leads to prolongation of circulation of said agent in the bloodstream.

Besides, a method for delivery of an agent to the organism, wherein:
i) at least one of the abovementioned substances are administered to the organism;
ii) before or after administration of said substance, or simultaneously with administration of said substance (co-administered with said substance), said agent is administered if it is not comprised in said substance.

Besides, a method characterized in that said agent mediates the diagnosis or therapeutic action.

Besides, a method characterized in that said agent is administered not less than 6 hours and not more than 18 hours after administration of said substance.

Besides, a method characterized in that said agent is administered not less than in 4, 7, 13, 30, 50, 70, 100, 125, 150, 200, 300, 400, 500, 600, 700, 800, 900 or 1000 hours after administration of said substance.

Besides, a method characterized in that said agent is administered not less than 11 hours and not more than 13 hours after administration of said substance.

Besides, a method characterized in that along with administration of said substance and said agent, a recovery preparation is administered to the organism; said recovery preparation causes (promotes) an increase (compared to the case without administration of said recovery preparation) of the number of cells or molecules, elimination of which from the bloodstream is stimulated (induced) by said component of said substance.

Besides, a method characterized in that said recovery preparation comprises the organism's blood or the organism's blood components, or donor's blood, or donor's blood components.

Besides, a method characterized in that said recovery preparation comprises erythropoietin.

Besides, a substance that is administered to the organism for increasing the time of circulation of the agent in the bloodstream; said substance comprising at least:
i) component 1 that, being administered to the organism, causes increased (intensifies, stimulates) elimination (clearance) from circulation in the bloodstream of erythrocytes or thrombocytes, which appeared in the bloodstream in a natural or artificial way, as compared with the rate of their elimination without administration of said component,
or ii) component 2 consisting of at least at least an antibody (or its variants), which is highly homologous to the antibodies of the organism, including autologous, allogeneic, humanized or chimeric antibody, capable of forming directly or indirectly a specific complex with the objects that are naturally present in the organism or have been brought (introduced) artificially (got in the organism artificially).

At this, said objects can be molecules, particles, cells, and their different variants or their combinations including complexes.

Besides, a substance characterized in that said antibody of said component 2 is gamma-immunoglobulin.

In one of the invention embodiments, said objects can be cells. Under the cells that appeared in the organism (or the bloodstream) in a natural way, the cells are meant, which have been naturally produced inside the organism and never left the organism, while under the cells, which are artificially brought, the cells are meant, which have been introduced into the organism, including the organism's cells which were extracted and then returned to the organism. The same is valid also for other objects, for example, molecules.

Besides, a substance characterized in that said erythrocytes or thrombocytes appeared in the bloodstream in an artificial way or said objects appeared in the bloodstream in an artificial way are introduced into the organism via transfusion of donor's or the organism's material.

In one of the invention embodiments, said material introduced into the organism via transfusion is donated blood, or the organism's blood or its components.

Besides, a substance characterized in that said erythrocytes or thrombocytes appeared in the bloodstream in an artificial way or said objects appeared in the bloodstream in an artificial way are a component of said substance.

Besides, a substance characterized in that said erythrocytes or thrombocytes, which appear in the bloodstream in an artificial way, or said objects, which appear in the bloodstream in an artificial way, are a part of said component 1 of said substance.

Besides, a substance characterized in that said erythrocytes or thrombocytes, which are forcefully eliminated from circulation in the bloodstream, are the processed variants of the organism's cells or allogeneic cells, which either are one of the substance components or appear in the organism in an artificial way.

Besides, a substance characterized in that said processing of the variants of the organism's cells or of allogeneic cells includes incubation with specific antibody, which directly or indirectly recognizes, or binds with said variants of the organism's cells or allogeneic cells.

Besides, a substance characterized in that said specific antibody is monoclonal antibody.

Besides, a substance characterized in that said objects are erythrocytes.

Besides, a substance characterized in that said objects are leucocytes.

Besides, a substance characterized in that said objects are thrombocytes.

Besides, a substance characterized in that said objects are albumins.

Besides, a substance characterized in that said objects are immunoglobulins.

Besides, a substance characterized in that said objects, which appear in the bloodstream in an artificial way, are objects introduced into the organism.

Besides, a substance characterized in that said component 1 or said component 2 causes (induces) mainly increased elimination (clearance) from said circulation of erythrocytes or thrombocytes, which have not left the organism.

Besides, a substance characterized in that said component 1 or said component 2 specifically interact with said erythrocytes or thrombocytes, or the objects, and said interaction causes (induces) increased elimination from circulation of said erythrocytes or thrombocytes, or objects.

Besides, a substance characterized in that said interaction of said component 1 or said component 2 with said erythrocytes or thrombocytes, or the objects, is binding of said component with said erythrocytes or thrombocytes, or the objects.

Besides, a substance characterized in that said component 1 or said component 2 non-covalently interact with the erythrocytes or thrombocytes, or the objects, which circulate in the bloodstream.

Besides, a substance characterized in that said binding of said component 1 or said component 2 with said erythrocytes or thrombocytes, or the objects is non-covalent.

Besides, a substance characterized in that said agent represents nanoparticles or microparticles (including particles comprising nanoparticles or microparticles).

Besides, a substance characterized in that said agent represents a nanoparticle or microparticle with size in one of the dimensions exceeding 10 nm.

Besides, a substance characterized in that said increased elimination of erythrocytes or thrombocytes is caused by their ageing under effect of said component 1.

Besides, a substance characterized in that said increased elimination of erythrocytes or thrombocytes, or objects is caused by their agglutination under effect of said component 1 or said component 2 of the substance.

Besides, a substance characterized in that said increased elimination of erythrocytes or thrombocytes is caused by their damage under effect of said component 1.

Besides, a substance characterized in that said component 1 or component 2 causes an increase of phagocytizing activity of the organism's cells, and this factor leads to the increased elimination of said erythrocytes or thrombocytes, or objects from circulation.

Besides, a substance characterized in that said prolongation of circulation of said agent is caused by at least partial blockade of the reticuloendothelial system due to elimination (clearance) of said erythrocytes or thrombocytes, or objects from circulation in the bloodstream.

Besides, a substance characterized in that said component 1 comprises at least an antibody (or its variants), which is highly homologous to the antibodies of the organism, including autologous, allogeneic, humanized or chimeric antibody., which are capable to specifically directly or indirectly recognize and bind to erythrocytes or thrombocytes.

Besides, a substance characterized in that said component 1 forms a specific complex with erythrocytes or thrombocytes through direct or indirect recognition and binding to said erythrocytes or thrombocytes.

Besides, a substance characterized in that said antibody of said component 2 forms said specific complex with said objects through direct or indirect recognition and binding to said objects.

Besides, a substance characterized in that said antibody is targeted against the organism's erythrocytes or their allogeneic analogs.

Besides, a substance characterized in that said antibody is targeted against the organism's thrombocytes or their allogeneic analogs.

Besides, a substance characterized in that said antibodies is targeted against the organism's leukocytes or their allogeneic analogs.

Besides, a substance characterized in that administration of said antibody causes agglutination of said objects.

Besides, a substance characterized in that said direct or indirect recognition and binding of said component 1 or said component 2 to erythrocytes or thrombocytes, or objects causes phagocytosis of said erythrocytes or thrombocytes, or objects by the organism's cells.

Besides, a substance characterized in that said direct or indirect recognition and binding of said component 1 or said component 2 to erythrocytes or thrombocytes, or objects causes mainly phagocytosis of said erythrocytes or thrombocytes, or objects by the organism's cells, said phagocytosis is mediated by Fc-receptor.

Besides, a substance characterized in that said component 1 or said component 2 is a chemical compound, a molecule, a particle or a cell (including a bacterium, leucocyte, etc.) or a combination of these entities, but said component does not comprise erythrocytes.

Besides, a substance characterized in that said component 1 or said component 2 is a chemical compound, a molecule, a particle or a cell (including a bacterium, leucocyte, etc.) or a combination of these entities, but said component does not comprise objects obtained by modification of erythrocytes.

Besides, a substance characterized in that said component 1 or said component 2 comprises at least several antibodies, which are highly homologous to the antibodies of the organism (or their variants), including autologous, allogeneic, humanized or chimeric antibodies.

Besides, a substance characterized in that said antibody of said component 2 is a monoclonal antibody or said component 1 comprises at least a monoclonal antibody.

Besides, a substance characterized in that said antibody of said component 2 is linked with other entities including molecules, particles, cells or their combinations, or in that said component 1 or said component 2 comprises at least antibody linked with other entities including molecules, particles, cells or their combinations.

Besides, a substance characterized in that said component 1 or said component 2 comprises at least several antibodies, which are highly homological to the species antibodies (or their variants) of the organism, including autologous, allogeneic, humanized or chimeric antibodies targeted at the organism's cells or molecules, or their allogeneic analogs.

Besides, a substance characterized in that said component 1 or said component 2 comprises at least a complex of an entity (including a molecule, cell, particle) with cells or molecules extracted (derived) from the organism or their allogeneic analogs.

Besides, a substance characterized in that said component 1 or said component 2 comprises at least a complex of molecules or cells extracted (derived) from the organism (or their allogeneic variants) and antibodies bound with them directly or indirectly.

Besides, a substance characterized in that said component 1 or said component 2 comprises at least a complex of molecules or cells extracted (derived) from the organism (or their allogeneic variants) and antibodies bound with them, said antibodies are highly homological to the species antibodies (or their variants) of the organism, including autologous, allogeneic, humanized or chimeric antibodies.

Besides, a substance characterized in that said component 1 or said component 2 comprises at least a complex of molecules or cells extracted (derived) from the organism (or their allogeneic variants) and antibodies to said molecules or cells (or to their variants), said antibodies are highly homological to the species antibodies (or their variants) of the organism, including autologous, allogeneic, humanized or chimeric antibodies.

Besides, a substance characterized in that said component 1 or said component 2 is a complex or complexes of cells or molecules extracted (derived) from the organism (or their allogeneic analogs) specifically bound with antibodies against erythrocytes or thrombocytes.

Besides, a substance characterized in that said component 1 or said component 2 comprises at least a complex of an entity (molecule, cell, particle, etc.), which is non-covalently bound via a specific biomolecular bond with an erythrocyte or thrombocyte extracted (derived) from the organism (or their allogeneic analog).

Besides, a substance characterized in that said component 1 or said component 2 comprises at least antibody, which is highly homologous to the antibodies of the organism, including autologous, allogeneic, humanized or chimeric antibody, which can specifically directly or indirectly recognize and bind to the cells or molecules that circulate in the bloodstream (naturally circulating or brought from outside).

Besides, a substance characterized in that said antibody, which can specifically recognize and bind directly or indirectly to the cells or molecules that circulate in the bloodstream, is a monoclonal antibody.

Besides, a substance characterized in that said antibody (or its variants), which can specifically directly or indirectly recognize and bind to the cells or molecules that circulate in the bloodstream, is linked (or bound) with other substances that comprise, inter alia, molecules, particles, cells or their combinations.

Besides, a substance characterized in that said antibody specifically interact with cells or molecules of the organism, and this interaction induces (leads to) increased elimination of said cells or molecules of the organism from circulation.

Besides, a substance characterized in that said component 1 or said component 2 comprises at least antibody, which is monoclonal or at least highly homologous to the organism's antibodies (or their combinations and variants), including autologous, allogeneic, humanized or chimeric antibodies, against cells or molecules of the organism or their allogeneic analogs. Certainly, here and in the other similar cases, it is implied that said antibody can be targeted at (specific for), for example, cell markers of said cells, definite epitopes of cell markers, etc.

Besides, a substance characterized in that said component 1 or said component 2 comprises at least several antibodies, which are monoclonal or at least homologous to the antibodies of the organism (or their combinations and variants), including autologous, allogeneic, humanized or chimeric antibodies, against cells or molecules of the organism or their allogeneic analogs.

Besides, a substance characterized in that said component 1 differs from an erythrocyte or a modified erythrocyte.

Besides, a substance, administration of which into the organism induces (leads to) said prolongation of circulation time of said agent in the bloodstream so that half-life of said agent's circulation increases not less than 3-fold as compared with half-life of said agent without administration of said substance.

Besides, a substance, administration of which into the organism induces (leads to) said prolongation of circulation time of said agent in the bloodstream so that the half-life of the agent's circulation in bloodstream increases not less increases not less than by 1.2 times, more preferably by 1.5 times, more preferably by 1.75 times, more preferably by 2 times, more preferably by 3 times, more preferably by 5 times, more preferably by 10 times, more preferably by 12.5 times, more preferably by 15 times, more preferably by 20 times and even more preferably by 25 times as compared with half-life of the agent's circulation without administration of the substance.

Besides, a substance, administration of which into the organism induces (leads to) said prolongation of circulation time of said agent in the bloodstream, and said prolongation of circulation time of said agent is temporary.

Besides, a substance, administration of which into the organism increases circulation time of said agent, administration of which is used for diagnostics or monitoring, or therapy of a disease, wherein said prolongation of circulation time of said agent enhances the efficiency of the diagnostics or therapy of disease.

Besides, a method for prolongation of circulation time of an agent in the bloodstream of the organism, wherein:
i) at least one of the abovementioned substances is introduced into [administered to] the organism;
ii) said agent is introduced into [administered to] said organism.

Besides, a method, wherein the agent administration is used for diagnostics or monitoring, or therapy, and the prolongation of circulation time of said agent in the bloodstream enhances the efficiency of the diagnostics or therapy.

Besides, a method characterized in that the dose of said substance to be administered to the organism is selected to increase circulation time by the required quantity of times (by the predetermined multiplier/factor) for optimization of the balance between the beneficial effects from the increased circulation times and possible negative effects (such as lowering the concentration of molecules or blood cells eliminated by the RES organs).

Besides, a method characterized in that the dose of said substance to be administered to the organism is selected to increase circulation time by the required quantity of times (by a predetermined multiplier/factor) for optimization of the balance between the beneficial effects from the increased circulation times and possible negative effects.

Besides, a method characterized in that administration of said substance or its variants is repeated several times.

Besides, a method characterized in that administration of said substance or its variants is repeated several times. Under the variant of said substance, a substance is understood that is different in composition from said substance, but is also the subject of this invention according to its features.

Besides, a method characterized in that along with administration of said agent and said substance, a recovery preparation (regenerating agent, regenerating preparation) is administered to the organism; said recovery preparation causes an increase of the amount of cells or molecules, whose elimination from circulation in the bloodstream is increased under the influence of said substance as compared with that amount without administration of said recovery preparation.

Besides, a method characterized in that along with administration of said agent and said substance, a recovery preparation is administered to the organism; said recovery preparation causes an increase of the amount of erythrocytes or thrombocytes in blood as compared with that amount without administration of said recovery preparation.

Besides, a method characterized in that said recovery preparation comprises blood of said organism or its components, or donated blood or its components.

Besides, a method characterized in that said recovery preparation comprises erythropoietin.

Besides, a substance administered to the organism with the purpose of enhancing the diagnostic or therapeutic effect of an agent introduced into the organism; said substance comprising at least a component, which, upon administration to the organism, induces (leads to) at least partial blockade of the reticuloendothelial system due to active elimination by the reticuloendothelial system from circulation in the bloodstream of at least one of the following entities:
i) own cells or molecules of the organism [cells or molecules of the organism itself]; or ii) cells or molecules introduced in the organism artificially but which are not comprised in said substance,
or iii) cells comprised in said substance, including those comprised in said component.

Besides, a substance for enhancing the diagnostic or therapeutic effect of an agent; said substance comprising at least a component, which, upon administration of said substance to the organism promotes elimination by the reticuloendothelial system from circulation in the bloodstream of at least objects, which are cells or molecules, if said molecules are not eliminated from the bloodstream primarily as opsonins, which interact with said component non-specifically; said elimination of said objects from circulation in the bloodstream induces (leads to) at least partial blockade of the reticuloendothelial system.

Besides, a substance administered to the organism with the purpose of enhancing the diagnostic or therapeutic effect of an agent; said substance comprising at least a component, which, upon administration to the organism, causes active elimination (promotes elimination) from circulation in the bloodstream of objects of the organism or objects introduced to the organism irrespective to administration of said substance, or cells comprised in said component.

Besides, a substance administered to the organism with the purpose of enhancing the diagnostic or therapeutic effect of an agent; said substance comprising at least a component, which, upon administration to the organism, causes active elimination (promotes elimination) from circulation in the bloodstream of cells of the organism or cells comprised in said component or objects introduced to the organism irrespective to administration of said substance.

Besides, a substance administered to the organism with the purpose of enhancing the diagnostic or therapeutic effect of an agent; said substance comprising at least a component, which, upon administration to the organism, causes at least partial blockade of the reticuloendothelial system with at least one of following entities: cells or molecules capable of forming a specific complex with said component.

Besides, a substance for enhancing the diagnostic or therapeutic effect of an agent; said substance comprising at least a component, which, upon administration of said substance to the organism promotes elimination from circulation in the bloodstream of objects, which comprise cells or molecules, if said molecules are not eliminated (cleared) from the bloodstream primarily as opsonins, which interact with said component non-specifically; said elimination (clearance) of said objects from circulation in the bloodstream induces (leads to) at least partial blockade of the reticuloendothelial system.

Besides, a substance administered to the organism for enhancement of the diagnostic or therapeutic performance of an agent, said substance comprising at least antibody, which is highly homologous to the organism's antibodies including autologous, allogeneic, humanized or chimeric antibody, against erythrocytes or thrombocytes (or their variants).

Besides, a substance administered to the organism for enhancement of the diagnostic or therapeutic performance of agent or for delivery of the agent, or prolongation of circulation of the agent in the bloodstream; said substance comprising at least antibody, which is highly homologous to the organism's antibodies including autologous, allogeneic, humanized or chimeric antibody, against erythrocytes or thrombocytes (or their variants).

Besides, a substance administered to the organism for enhancement of the diagnostic or therapeutic performance of agent and comprising at least antibody, which is highly homologous to the organism's antibodies including autologous, allogeneic, humanized or chimeric antibody, against erythrocytes or thrombocytes (or their variants).

Besides, a substance for delivery of an agent to the organism and comprising at least antibody, which is highly homologous to the organism's antibodies including autologous, allogeneic, humanized or chimeric antibody, against erythrocytes or thrombocytes (or their variants).

Besides, a substance for increasing the circulation time of an agent in the bloodstream and comprising at least antibody, which is highly homologous to the organism's antibodies including autologous, allogeneic, humanized or chimeric antibody, against erythrocytes or thrombocytes (or their variants).

Besides, a method for increasing the circulation time of an agent in the bloodstream of the organism by administration of said agent to said organism along with the substance, administration of which induces (leads to, stimulates) increased elimination from circulation in the bloodstream of erythrocytes or thrombocytes as compared with the rate of their elimination without administration of said substance, said substance being different from erythrocytes.

Besides, a method for increasing the circulation time of an agent in the bloodstream of the organism by administration of said agent to said living organism along with the substance, administration of which leads to increased elimination from circulation in the bloodstream of erythrocytes or thrombocytes as compared with the rate of their elimination without administration of said substance, said substance being different from erythrocytes modified in any way, including the disintegrated erythrocytes.

Besides, a method for promoting products to the market, comprising at least promotion for diagnostics, monitoring or therapy of diseases or conditions of the organism, or for scientific research of at least one of the abovementioned substances or methods.

Besides, a method characterized in that the promotion is carried out by using a leaflet inserted into the package with a commercial substance, comprising at least one of the abovementioned substances.

Besides, a method characterized in that the promotion is carried out by written or oral communication to a doctor or provider of medical services.

Besides, a business method, comprising at least marketing of at least one of the abovementioned substances and methods for the purpose of diagnostics and therapy, or enhancement of efficiency of diagnostics or therapy, or for delivery of an agent to the organism, or for increase of the circulation time of the agent in the bloodstream.

Besides, to achieve said technical result, substances and methods featuring the combinations of the abovementioned characteristics are proposed. Besides, in all cases, mentioning, e.g., of cells or molecules means cells or molecules of the organism, or allogeneic cells, or variants of the organism's cells or allogeneic cells.

All embodiments of the invention listed below are given solely for illustration of the diversity of the invention variants rather than as limitations.

In one embodiment of the invention, prolongation of the agent circulation in the bloodstream of the organism or blockade of the RES are achieved through administration to said organism of a substance, which causes increased (intensified) elimination from circulation in the bloodstream of native (or intact) erythrocytes or thrombocytes of the organism as compared with the rate of their elimination without administration of said substance. Under native erythrocytes or thrombocytes, the unchanged cells are meant, which have not been subjected to hemolysis or any physical, chemical or biochemical processing (such processing can be, for example, ultrasound treatment, osmotic lysis, fixation with formalin, etc.). Accordingly, in one embodiment of the invention, elimination of said native cells is mediated by biomolecules that non-covalently interact with the cells.

In one embodiment of the invention, said substance is chosen so that it does not contain erythrocytes or, in a more general case, modified erythrocytes. In another embodiment of the invention, the components of said substance interact with erythrocytes or thrombocytes, or other cells or molecules of the organism, which are directly inside the organism meaning those, which are not brought to the organism within this manipulation with the organism (i.e. if the organism was earlier subjected to transfusion or transplantation not related to the need of modulation of the agent behavior in the organism, said cells or molecules can be also considered). Under the modified erythrocytes, the entities (substances, matter, preparations) are meant, which are obtained through any physical, chemical or biochemical processing of erythrocytes or blood components, which contain erythrocytes. Such processing can be, for example, ultrasound treatment, osmotic lysis, fixation with formalin, etc. Such modifications can lead to adverse toxic effects of these agents.

Besides, in one embodiment of the invention, said substance may contain complexes of pre-collected erythrocytes or other cells, or molecules of the organism with complementary antibodies, which are homological to the organism's antibodies, including autologous, allogeneic, humanized or chimeric antibodies. The substance that contains such complexes will not cause, for example, anemia, and the administered cells or molecules will be eliminated by the RES organs, and this will lead to blockade of the RES organs.

In another embodiment of the invention, such complexes may be formed using other specific entities (molecules, preparations, substances) instead of antibodies. It is important that when using non-covalently binding molecules (entities, preparations, substances), no new covalent bonds are formed, which can lead to undesirable toxic effects of these molecules (entities, preparations, substances). For example, in one embodiment of the invention lectins are used instead of antibodies; the lectins can, inter alia, agglutinate erythrocytes or other cells and lead also to their accelerated elimination from the bloodstream by the RES cells. The collection (harvesting) of cells or molecules of the organism can be performed in advance so that the organism could restore the levels of said substances by the moment of administration of the agent and substance. Besides, one can not only wait until the organism restores on its own the levels of said substances but actively facilitate the process by administration of recovery preparation. Said recovery preparation can be, for example, erythropoietin that accelerates erythropoiesis. Another variant of administration of the recovery preparation can be transfusion of donor blood. Administration of the recovery preparation can be carried out before or after, or simultaneously with administration of the agent and substance (co-administered with the agent and substance).

In one embodiment of the invention, elimination of cells from the bloodstream is facilitated by their treatment with enzymes. Said treatment of erythrocytes with enzymes, for example, e.g., neuraminidase, trypsin, galactose oxidase, etc. or a combination of enzymes induces (leads to) accelerated elimination of the erythrocytes from circulation in the bloodstream by the reticuloendothelial system. This is due to, for example, altering the glycosylation profile of the erythrocyte surface. That can be detected (recognized) by the RES cells, which eliminate the "aged" erythrocytes. Appearance on the erythrocyte of new antigen determinants will also cause its binding with circulating antibodies and elimination from the bloodstream by, for example, phagocytosis mediated by a Fc-receptor. It is evident that although the treatment of the cells with enzymes has certain advantages due to availability of enzymes, the treatment of the cells with antibody provides milder effect on the organism (in terms of toxicity - due to possibility of appearance of new antigen determinants, reproducibility, etc.)

In any of the mentioned embodiments of the invention, the active component or components of said substance can comprise a combination of different objects, including those, which possess the described properties, and those, which do not. Namely, the substance can comprise not only, for example, allogeneic antibody against erythrocytes but also various auxiliary components such as buffer salts, etc. Besides, said antibody can be conjugated, for example, with other molecules, particles, etc.

Under the cells and molecules of the organism, the cells and molecules of the organism itself are meant as well as, for example, molecules formed by the organism as a result of food digestion, etc.

A wide variety of entities can be employed as the agent, e.g., nanoparticles or microparticles in the usual sense of colloid chemistry, bacteria or cells (in the preferred embodiment of the invention), diverse molecules (in the preferred embodiment - macromolecules), macromolecular complexes, etc. The agents may be, inter alia, diagnostic and/or therapeutic (including theranostic) agents or entities (substances, preparations), which mediate diagnostic and/or therapeutic functions in one way or another. For example, the agent can perform visualization function for diagnostics or monitoring of the condition/disease of the organism; for example, this can be the agent that directly generates the detectible signal (fluorescent, luminescent or radioactive agent), or that changes the signal detected from other entities (substances, preparations, matte) (e.g., from water protons for MRI contrasting agents). Aside from the physical signals, the agent may participate in generation of biochemical signals that, for example, permit one to test the organism for a disease and, if necessary, to signal about it. In addition, the agent may also perform other functions (including the therapeutic ones), for example, for directed drug delivery, for hyperthermia effect on the organism (if it, for example, contains magnetic nanoparticles), photodynamic therapy (causing, one way or another, for example, cytotoxicity under the influence of external radiation), etc.

The ability of an entity to form, directly or indirectly, a specific complex with something, for example, with the organism's cells means that either said entity (for example, antibody) can directly recognize and bind to a cell or molecule, or recognize and bind to other additional molecules, which, in turn, can (also directly or via other molecules) bind to said organism's cells. Said additional molecules can be created by the organism itself, or administered to the organism, including administration as part of the substance. Besides, under the indirect recognition and binding it can be understood that an antibody, for example, can bind not directly to the cell, but to another molecule bound with the cell. For example, a humanized antibody against rat immunoglobulins G can recognize and bind to rat antibody against human erythrocytes associated with (bound with) erythrocytes.

In all the abovementioned and following cases, under the blood circulation (circulation in the bloodstream or blood flow) one can understand the circulation in the bloodstream in general (in the "spatial" sense, i.e., inside the bloodstream, blood vessels, capillaries, etc.), as well as free circulation in the bloodstream (i.e., free circulation, for example, if an erythrocyte is absorbed by a macrophage circulating in the bloodstream, the erythrocyte is considered eliminated from circulation in the bloodstream, even though it is still inside the blood vessel in the spatial sense). In the preferable embodiment of the invention, the elimination (clearance) from circulation refers to elimination from free circulation.

In one embodiment of the invention, a short-term reversible refocusing of the RES on own cells/molecule of the organism is achieved in order to prevent the RES "attack" on the agent; that is fundamentally different from the known methods of RES blockade because the dose of administered foreign agent can be reduced 10-100 times as compared to existing methods to achieve 1000% increase of half-life of the agent (compared to circulation time of the agent without administration of any additional substances (preparations)). That is probably because most of the mass/volume of the resulting complexes, which are eliminated by the RES organs and cause blockade of the RES organs, belongs to the own blood components (components of the organism itself) and is a natural own material for the organism. That also permits minimization of the method toxicity because only a small amount of foreign (and therefore potentially toxic) agent is required to produce the desired effect.

In one embodiment of the invention, a substance is used that comprises at least a component that comprises at least an antibody (or its variants), which is homologous to the antibodies of the organism, including autologous, allogeneic, humanized or chimeric antibody, capable of forming directly or indirectly a specific complex with the cells or molecules that are naturally present in or are artificially brought to the organism. In another embodiment of the invention, an antibody (or its variants), which is highly homologous to the antibodies of the organism, including autologous, allogeneic, humanized or chimeric antibody, against the blood cells of the patient (organism, subject), for example, his/her erythrocytes, thrombocytes or leucocytes, is administered to induce blockade of the RES organs. In this case, the RES organs actively ingest (eliminate, clear, perform clearance of) the blood cells bound with the antibody, after that their ingestion activity against the agent decreases; in particular, this can increase the half-life of the agent's circulation in the bloodstream. After administration of the antibody, the agent circulation prolongation effect increases during certain time period, and then decreases. In one embodiment of the invention, if the agent is administered after antibody administration, the half-life of the agent's circulation will be increasing during certain time period, and then will decrease to the value observed before the antibody administration (see Examples 2-3). In the embodiment with antibody against erythrocytes, the induced anemia (due to hematocrit decrease) is short-term (non-permanent), and the amount of erythrocytes in the bloodstream usually restores to its level observed before the antibody administration.

The administration of antibody, which is highly homologous to the antibodies (or their variants) of the organism, including autologous, allogeneic, humanized or chimeric antibody, rather than, for example, heterologous anti-lymphocyte serum to block the RES permits engaging of only natural mechanism of elimination of cells from the bloodstream by phagocytosis by the RES cells mediated by Fc-receptor and does not introduce unnecessary risk (toxicity) as a result of, for example, "attack" of the phagocytes by proteins of anti-lymphocyte serum. In a preferable embodiment of the invention, the Fc-fragment of antibody is identical to the Fc-fragment of the organism's antibodies. Besides, the high homology of the antibody to the organism's antibodies will allow minimizing the immune response to components of the substance or avoiding such response (making them non-immunogenic), and that will permit multiple (repetitive) injections of said substance to achieve the desired effect. In addition, this will minimize the incidence of various side effects from the immune response, such as inability to carry out blood immunoassays, which employ certain antibodies (for example, if a mouse antibody against human erythrocytes is administered to a subject, and, as an immune response, antibodies against mouse immunoglobulin are formed in the subject's organism, many tests for blood immunoassays that involve murine antibodies will be difficult to conduct because of high probability of errors in the test results).

Besides, since the number of erythrocytes in blood many times exceeds the number of leukocytes and thrombocytes, the use of substances for inducing RES clearance of erythrocytes for the RES blockade, including for the purposes of increasing blood circulation half-life of the agent, is preferable because of lesser negative effects on the organism's health. In particular, after elimination by the RES of a certain portion of erythrocytes from the bloodstream, in one embodiment of the invention, hematocrit is restored by blood transfusion. This way, negative consequences of anemia associated with reduced hematocrit can be avoided.

One of the invention embodiments (when components of the own blood are eliminated by the RES cells) is characterized by low toxicity because RES clearance of blood components (including erythrocytes) occurs continuously throughout the whole life of mammals, and this process is only briefly accelerated in the proposed invention. For example, during 24 hours in the natural conditions, the body processes about 1% of erythrocytes, while for the RES blockade it is necessary to eliminate from the bloodstream less than 10% of erythrocytes (in Example 2, the maximum drop in hematocrit was 10-15%, i.e., from 50% to 45%, moreover, hematocrit minimum was observed not in the first 24 hours); the blockade effect lasts about 5-10 days, during which approximately the same number of erythrocytes would be processed. For that reason, this method features low risks for health. Besides, for example, when antibody against blood cells is applied to increase the circulation time of the agent, a natural mechanism is used to eliminate cells from the bloodstream by the RES cells. In particular, the antibody binds non-covalently to the cell and signals to the RES cells that this complex should be eliminated (cleared) from the bloodstream. An important advantage of the method is that no chemical modification of the blood molecules/cells occurs, no new covalent bonds or new chemical compounds are formed, etc., which could cause any toxic effects.

In one embodiment of the invention, an antibody (and/or antibody dose) is chosen, which does not lead to activation of the complement system or leads to insignificant activation of the complement system; said antibody (and/or antibody dose) predominantly promoting Fc-receptor-mediated phagocytosis by the RES cells.

Besides, in one embodiment of the invention, the substance does not cause agglutination of blood cells. In this case, the dose of the administered agent can vary within a wide range without a risk of vascular embolization.

Besides, in one embodiment of the invention, the substance components, on the contrary, cause agglutination of the blood cells. In that case, a dose of substance should be selected to prevent thrombosis, which results in the vessels embolization.

Besides, in one embodiment of the invention, the dose of the administered substance is selected (adjusted) to increase the effect of the RES blockade or to increase the circulation time of the agent in the bloodstream as necessary (by predetermined multiplier/factor) to optimize the balance between the advantageous effects (outcome) of the increase of the circulation time and possible negative effects of the decrease of concentration of blood molecules/cells eliminated by the RES.

This approach can also be used to block phagocytic cells of the RES in other natural fluids, as well as in the organism's tissues. Upon administration of corresponding entities that cause phagocytic cells of the RES to actively ingest (perform clearance of) cells and/or molecules of the organism, or other objects, their ability to phagocytize the agent decreases, and that leads to increased target efficacy of the agent.

The present invention can be used for diagnostic and/or therapeutic purposes, including for enhancing the targeted delivery of various agents to different targets in the organism. The nature and type of the targets can be most diverse, for example, it can be specific cells, tissues, areas of the organism (including malignant neoplasms, tumors and inflammation sites).

The present invention can be used, for example, as follows. A substance that causes the RES blockade (e.g., humanized antibody against erythrocytes) is intravenously administered to the organism; then polymer particles containing toxic molecules (e.g., an anticancer drug doxorubicin or cisplatin) are intravenously injected. Because of enhanced permeability and retention effect, or due to specific anti-cancer antibodies, the particles accumulate in the tumor (as shown in Example 6), where doxorubicin or cisplatin is desorbed and causes the death of the tumor cells.

In one embodiment of the invention, a monoclonal antibody, preferably against erythrocytes or thrombocytes, or, more preferably, bound with allogeneic erythrocytes or thrombocytes, preferably with erythrocytes or thrombocytes of the organism or their allogeneic variants, is used. At this, in one embodiment of the invention, a monoclonal antibody is chosen by its type and isotype so that to achieve controllable elimination of cells from circulation primarily according to a definite mechanism, for example, due to phagocytosis of the cells, to which the antibody binds, according to the Fc-receptor-mediated mechanism (to block, for example, the Kupffer cells of the organism), or agglutination of erythrocytes for filtration of aggregates by the spleen, due to complement-mediated hemolysis of cells for increasing the kidney load, etc. In one embodiment of the invention, a combination of different antibodies is used to influence the biodistribution of the eliminated (cleared) cells or other objects that block the RES cells, as well as the biodistribution of the agent over the organism's tissues. For example, in the case of predominant blockade of liver, the agent will mainly accumulate in the target (e.g., in a tumor), as well as in the spleen, lungs, etc. Besides, the use of monoclonal antibodies allows creation of a substance of accurate and highly reproducible composition, which is useful for quality control and minimizing harmful effects from administration of said substance.

In one of the invention embodiments, a humanized or chimeric antibody preferably against erythrocytes or thrombocytes, or, more preferably, bound with erythrocytes or thrombocytes, preferably with erythrocytes or thrombocytes of the organism or their allogeneic variants, is used. Besides, fabrication of a monoclonal antibody, for example, of mouse or rat, against, for example, human erythrocytes, and its following humanization, permit obtaining of a high affinity antibody of the desired isotype with the desired Fc fragment, etc. It is also possible to get a desired antibody variant using the phage display technique.

In one embodiment of the invention, the substance contains a component that induces (leads to) increased elimination from circulation of the organism's molecules, mainly macromolecules or macromolecule complexes. This is important, inter alia, for full blockade of the cells that can eliminate the agents from circulation in the bloodstream, mostly for blockade of all types of such cells. This can significantly reduce the dose of the agent to be administered to deliver a certain quantity of the agent to target and improve the specificity of the delivery, i.e., to deliver to a target as much amount of the administered dose of the agent as possible.

In one of the invention embodiments, an antibody or a mixture of antibodies against simultaneously erythrocytes, thrombocytes and leucocytes (including those bound to erythrocytes, leucocytes and thrombocytes), preferably with the components that promote increased elimination from the organism of various molecules (preferably, macromolecules or macromolecule complexes) is used. That permits blockade of major part of the cells capable of elimination of the agent from circulation in the bloodstream and, thus, allows reducing the required dose of the agent.

### Operation of the proposed method is illustrated by drawings in Figs. 1-3.

List of drawings:
Fig. 1. Blood circulation dynamics of magnetic particles administered to the bloodstream of a mouse recorded as described in Example 1. The particles were administered after approximately 1.2 minutes after the start of the dynamics recording.
Fig. 2. Exponential fitting of the center part of the magnetic particles dynamics (the segment shown in Fig. 1 from 2nd min to ∼6.3 min) for determining the blood circulation half-life of the particles.
Fig. 3. The average content of magnetic particles in tumors of mice, which were injected with the magnetic particles after administration of the antibody and without administration of the antibody as described in Example 6.

### Examples

The variants of the invention implementation are diverse. Various examples are described below. The following examples are given only for illustration purposes and do not limit the applications of the invention.

**Example 1.** Measuring the circulation time (half-life) of magnetic particles in the bloodstream of a mouse.

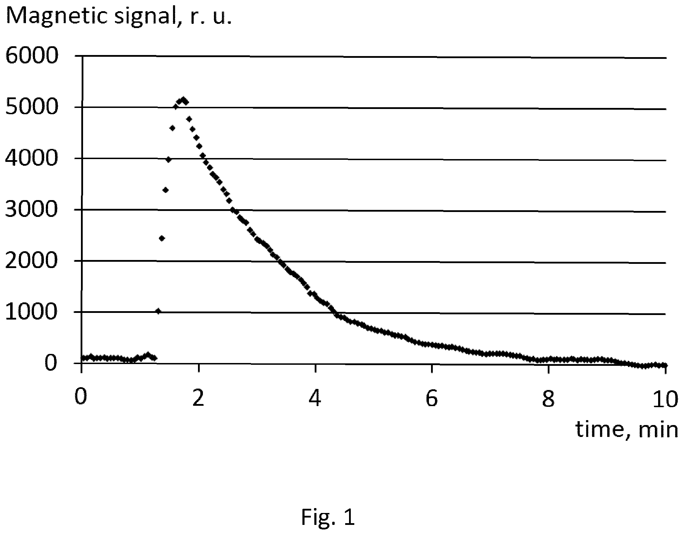

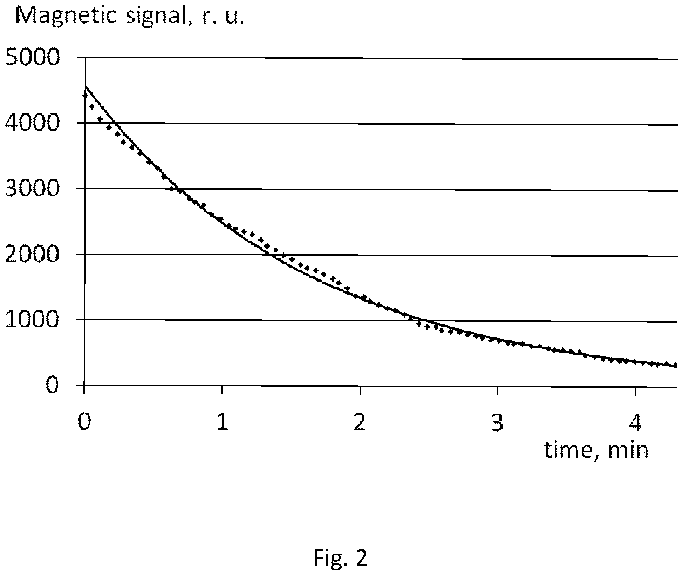

A Balb/c mouse tail is placed and fixed inside a coil of a magnetic nanoparticle detector created by the author of the invention described earlier in [Nikitin P.I., Vetoshko P.M. Magnetic Susceptibility Meter. Patent of Russian Federation 2177611 (2000)]. Then 100 µl of magnetic particle suspension was injected to retroorbital sinus of the mouse, and the dynamics of magnetic particle content in the bloodstream of tail veins and arteries of the mouse was recorded (see Fig. 1). The half-life of the particles' circulation in the bloodstream was calculated using either exponential fitting of the recorded data (see Fig. 2), or as the average time required for 2-fold decrease of the signal in the central part of the curve.

**Example 2.** Prolonging the circulation of nanoparticle- or microparticle-based agents in bloodstream of a mouse.

A dose of 1.3-3 mg/kg of a solution of monoclonal mouse antibody against mouse erythrocytes (34-3c clone) (or equal volume of 100 µl of physiological saline in a control experiment) was administered to Balb/c mice intraperitoneally or into the retroorbital sinus. Then, after 10-15 h, magnetic particles with characteristic size of 100 nm (hydrodynamic diameter), or 240 nm, or 1 µm were administered into retroorbital sinus, and the dynamics of particle concentration in the bloodstream was recorded as described in Example 1. On average, the half-life values of the particles after antibody administration were 9 times longer for 100-nm particles, 26 times longer for 240-nm particles, and 10 times longer for 1-µm particles as compared with the values measured in the control experiments.

**Example 3.** Prolonging the blood circulation of nanoparticle and microparticle agents in the mouse by a complex of antibody and cells.

A solution of monoclonal mouse antibody against mouse erythrocytes (34-3c clone) (or equal volume of physiologic saline in a control experiment) was incubated for 1 h with erythrocytes extracted from 100-200 µl of mouse blood (thrice washed by centrifuging with phosphate buffered saline). The so obtained complexes of erythrocytes and antibody were administered to Balb/c mice into a retroorbital sinus. Then, after 10 hours, magnetic particles with characteristic size of 100 nm (hydrodynamic diameter), or 240 nm, or 1 µm were administered into the retroorbital sinus, and the dynamics of particle concentration in the bloodstream was recorded as described in Example 1. The half-life of the particles' circulation after administration of the antibody complexes was 3-22 times longer as compared with that obtained in the control experiments.

**Example 4.** Reversibility of the effect of increased circulation time of nanoagents and microagents in the mouse bloodstream.

A dose of 1.5 mg/kg of solution of monoclonal mouse antibody against mouse erythrocytes (34-3c clone) (or equal volume of physiologic saline in a control experiment) was administered to Balb/c mice intraperitoneally. Then, after 10 days, magnetic particles with characteristic dimension of 100 nm (hydrodynamic diameter), or 240 nm, or 1 µm were administered into retroorbital sinus, and the dynamics of particle concentration in the bloodstream was recorded as described in Example 1. The half-life of the particles after antibody administration did not significantly differ (i.e, was within the experimental error) from that obtained in the control experiments.

**Example 5.** Enhancing the efficiency of targeted delivery of an agent.

A dose of 1.5 mg/kg of solution of monoclonal mouse antibody against mouse erythrocytes (34-3c clone) (or equal volume of physiologic saline in a control experiment) was administered to Balb/c mice intraperitoneally. Then, after 10-15 h, magnetic particles of characteristic size of 240 nm conjugated with fluorescent-labeled anti-mouse CD4 (cell marker of T-cells) were administered into retroorbital sinus. 30 min later, a blood sample was taken from the retroorbital sinus and stained with the anti-mouse CD4 antibody labeled with another fluorescent dye. The percentage of cells stained with both mentioned dyes was quantified by flow cytometry. In the case when antibodies against erythrocytes were administered to mice, the mean staining (mean fluorescence) of CD4 + cells with the first dye (and, consequently, with the particles) was 3-9 times higher than that in the case of administration of physiological saline.

**Example 6.** Enhancing the efficiency of delivery of an agent to a tumor.

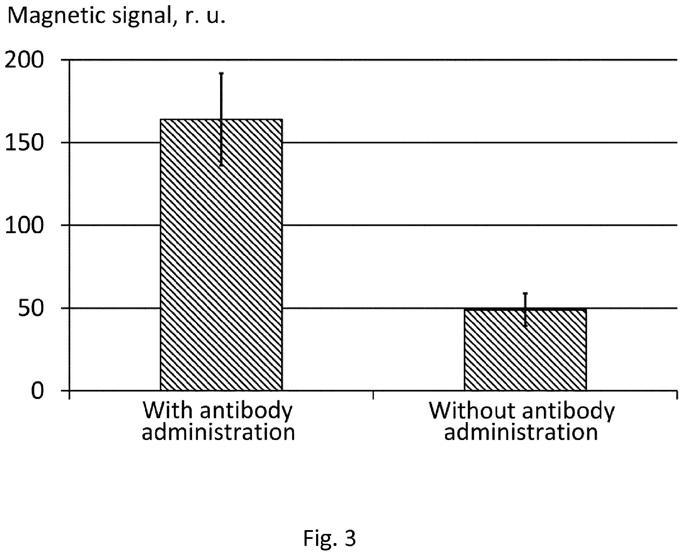

Mice were subcutaneously inoculated with 10^6 of B16F10 melanoma cells. After tumor development, an agent (nanoparticles and microparticles) was administered to the mice having 150-200 mm³ tumors as described in Example 2 (after administration and without administration of a solution of monoclonal mouse antibody against mouse erythrocytes). After 5 h since administration of the particles, the organs and tumor were extracted. The content of magnetic particles in the tumor was measured by a detector of magnetic nanoparticles developed by the author of the present invention and described earlier in [Nikitin P.I., Vetoshko P.M. Magnetic Susceptibility Meter. Patent of Russian Federation 2177611 (2000)]. The particle content (per tissue gram) in mouse tumors was 2-3 times higher after administration of anti-erythrocyte antibody than the particle content in the mouse tumors in the cases without administration of the anti-erythrocyte antibody (see the difference in Fig. 3 for 100-nm particles).

**Example 7.** Enhancing the efficiency of targeted delivery of an agent by means of preliminary administration of anti-erythrocyte xenogeneic antibody.

The dose of 50 mg/kg of a solution of polyclonal rabbit antibody (gamma-immunoglobulins) against mouse erythrocytes (or the equal volume of physiologic saline in the control experiments) was administered to Balb/c mice to retroorbital sinus. Then, after 10 h, the mice of the first group were injected to retroorbital sinus with magnetic particles with characteristic size of 240 nm, and the dynamics of particle concentration in the bloodstream was recorded as described in Example 1. The half-life of the particles' circulation after administration of the antibody increased 9-fold as compared with that measured after administration of physiologic saline. The mice of the second group after 12 h since the antibody administration were injected to retroorbital sinus with magnetic particles with characteristic size of 240 nm conjugated with fluorescent-labeled antibody against mouse CD4 (cell marker of T-cells). 30 min later, a blood sample was taken from the retroorbital sinus and stained with the anti-mouse CD4 antibody labeled with another fluorescent dye. The percentage of cells stained with both mentioned dyes was measured by flow cytometry. In the case when antibodies against erythrocytes were administered to mice, the mean staining (mean fluorescence) of CD4 + cells with the first dye (and, consequently, with the particles) was 2.7 times higher than that in the case of administration of physiological saline.

**Example 8.** Enhancing the efficiency of delivery of an agent to a tumor by means of preliminary administration of anti-erythrocyte xenoantibody.

Mice were subcutaneously inoculated with 10^6 of B16F10 melanoma cells. After development of the tumors, an agent (50-nm magnetic nanoparticles) was administered to the mice having tumors of 50-200 mm³ in 12 h after administration of 50 mg/kg of solution of polyclonal rabbit gamma-immunoglobulin against mouse erythrocytes and without administration of these antibody. After 5 h since administration of the particles, the organs and tumor were extracted. The content of magnetic particles in the tumor was measured by a detector of magnetic nanoparticles as in Example 6. The particle content (per tissue gram) in mouse tumors was 1.7 times (on average) higher after administration of anti-erythrocyte antibody than the particle content in the mouse tumors in the case without administration of the anti-erythrocyte antibody.

**Example 9.** Increasing the circulation time of of nanoagents and microagents in the mouse bloodstream by means of administration of xenogeneic antibody and secondary allogeneic antibody.

The 10 mg/kg dose of a solution of polyclonal rabbit antibody (gamma-immunoglobulins) against mouse erythrocytes (or the equal volume of 100 µl of physiologic saline in a control experiment) was intravenously administered to Balb/c mice. After 1 h, the mice were intravenously injected with a solution of polyclonal mouse antibody against rabbit gamma-immunoglobulins (or the equal volume of 100 µl of physiologic saline in a control experiment) in the dose of 1.3 - 3 mg/kg. Then, after 10-15 h, magnetic particles with characteristic dimension of 100 nm (hydrodynamic diameter) were administered into retroorbital sinus, and the dynamics of particle concentration in the bloodstream was recorded as described in Example 1. The half-life of the particles after administration of the rabbit and mouse antibodies was on average 2.8 times longer as compared with those obtained in the control experiments (only one type of antibody or without antibody administration).

**Example 10.** Increasing the life-span of animals with melanoma.

Mice were subcutaneously inoculated with 10^6 of B16F10 melanoma cells. After development of the tumors, an agent (polymer nanoparticles based on Poly Lactic-co-Glycolic Acid polymers) obtained via emulsification method with encapsulated doxorubicin) was administered to the mice having tumors of 50-200 mm³ as described in Example 2 after administration and without administration of a solution of monoclonal mouse antibody against mouse erythrocytes. An average life-span of the animals in the group that received the agent with doxorubicin after administration of the monoclonal mouse antibody against mouse erythrocytes proved to be 20-25% longer than the average life-span of the animals in the group that received solely the agent.

## Claims

1. A substance for enhancing diagnostic or therapeutic efficacy of an agent administered to the organism, said substance comprising at least a component, which, upon administering of the substance to the organism, induces clearance from the bloodstream by the reticuloendothelial system of at least objects, which circulate in the bloodstream but do not represent artificially created nanoparticles or microparticles, or opsonins that non-specifically bind to said component,
wherein said clearance of the objects causes at least partial blockade of the reticuloendothelial system.

2. The substance of claim 1, **characterized in that** said objects are cells.

3. The substance of claim 1, **characterized in that** said objects are erythrocytes.

4. The substance of claim 1, **characterized in that** said objects are thrombocytes.

5. The substance of claim 1, **characterized in that** said objects are leucocytes.

6. The substance of claim 1, **characterized in that** said objects are molecules.

7. The substance of claim 1, **characterized in that** said objects are a part of the substance but are not comprised in the composition of the component.

8. The substance of claim 1, **characterized in that** said objects are cells or molecules of the organism itself.

9. The substance of claim 1, **characterized in that** said objects are eliminated from free circulation in the bloodstream.

10. The substance of claim 1, **characterized in that** said objects are cells that have not left the organism.

11. The substance of claim 1, **characterized in that** said objects are cells or molecules that have been got to the organism artificially.

12. The substance of claim 1, **characterized in that** said objects are cells or molecules that have been got to the organism by transfusion of blood or donor blood constituents (components).

13. The substance of claim 1, **characterized in that** said component specifically interacts with said objects, and said interaction results in said elimination by the reticuloendothelial system.

14. The substance of claim 13, **characterized in that** said interaction of said component with said objects is the binding of the component to said objects.

15. The substance of claim 1, **characterized in that** said component noncovalently interacts with said objects.

16. The substance of claim 1, **characterized in that** said component forms a specific complex with said objects due to direct or indirect recognition and binding to said objects.

17. The substance of claim 16, **characterized in that** said component comprises at least an antibody, which forms said specific complex with said objects due to direct or indirect recognition and binding to said objects.

18. The substance of claim 17, **characterized in that** said direct or indirect recognition and binding to said objects causes primarily the Fc-receptor-mediated phagocytosis of said objects by the organism cells.

19. The substance of claim 17, **characterized in that** said antibodies are targeted against erythrocytes or thrombocytes of the organism or allogeneic variants thereof.

20. The substance of claim 1, **characterized in that** said component comprises at least an antibody (or a variant thereof), which is highly homologous to the species antibodies of the organism, including autologous, allogeneic, humanized or chimeric antibody.

21. The substance of claim 1, **characterized in that** said component comprises at least a monoclonal antibody.

22. The substance of claims 20, 21, **characterized in that** said component induces elimination of said objects, which are the cells comprised in the substance composition, due to recognition of said cells and stimulation of phagocytosis thereof by the reticuloendothelial system.

23. The substance of claim 1, **characterized in that** said component comprises at least several antibodies targeted at several different types of cells or molecules of the organism.

24. The substance of claim 1, **characterized in that** said elimination of said objects by the reticuloendothelial system is caused due to agglutination of said objects under effect of said component of the substance.

25. The substance of claim 1, **characterized in that** said component causes enhancement in the phagocyting activity of the cells of the reticuloendothelial system of the organism, and said cells eliminate said objects from the bloodstream.

26. The substance of claim 1, **characterized in that** said component is a chemical compound, molecule, particle or cell (including bacteria, leukocyte, thrombocyte) or a combination of such objects, but does not comprise any objects obtained through any modification of erythrocytes.

27. The substance of claim 1, the administration of which results in said blockade of the reticuloendothelial system so that the half-life of the agent's circulation in the bloodstream increases at least 3 times as compared to the half-life of the agent's circulation without administration of the substance.

28. The substance of claim 1, **characterized in that** the mass of said component does not exceed 20% of the mass of the objects, which cause the blockade of the reticuloendothelial system.

29. The substance of claim 1, **characterized in that** the dose of active components, which induce the blockade of the reticuloendothelial system, does not exceed 5 mg per kg of the weight of the organism.

30. The substance of claim 1, administration of which to the organism enhances the efficiency of active delivery of said agent to a target by at least 3 times as compared to the agent's delivery to the target without administration of the substance.

31. The substance of claim 1, administration of which to the organism enhances diagnostic or therapeutic efficacy of said agent by at least 2 times as compared to those in the case of the agent's delivery to target without administration of the substance.

32. The substance of claim 1, **characterized in that** said agent comprises, inter alia, at least one of the following nanoparticles or microparticles: magnetic, fluorescent, protein (including a cross-linked, polymerized or aggregated protein), polymeric, including that consisting of at least one of the following polymers: polystyrene, dextran, polypeptide, polylactide glycolic acid, or block-copolymers) or crystalline (gold, silver, semiconductor, or metallic) nanoparticle or microparticle.

33. The substance of claim 1, **characterized in that** said agent is used for diagnostics or therapy of diseases or conditions of the organism, comprising one or more diseases from the following list: cancer, atherosclerosis, stroke, myocardial infarction, including diagnostics of diseases or conditions of the organism using angiocontrast examination.

34. The substance of claim 1, **characterized in that** said agent performs an imaging function due to generation of a detectable signal, including at least one of the following signals: fluorescent, luminescent, PET-signal, MRI-contrasting signal, X-ray contrasting signal, magnetic signal, or plasmonic resonance signal, or a signal generated due to absorption of light or other electromagnetic or acoustic waves.

35. The substance of claim 1, **characterized in that** said agent performs a therapeutic function, e.g., by delivering to targets the cytostatic or cytotoxic compounds, or medicinal compounds, including low-molecular, enzymatic, radioactive, chemotherapeutic compounds for photodynamic therapy, hyperthermia.

36. The substance for enhancing the diagnostic or therapeutic efficiency of an agent the administered to the organism, said substance comprises at least one component which, upon administration to the organism, induces at least partial blockade of the reticuloendothelial system with cells.

37. The substance of claim 36, **characterized in that** said component comprises at least an antibody against said cells, which provide said blockade of the reticuloendothelial system; said antibody being highly homologous to the antibodies of the organism, including autologous, allogeneic, humanized or chimeric antibody.

38. The substance of claim 36, **characterized in that** said component leads to the enhanced elimination by the reticuloendothelial system of the cells circulating in the bloodstream, thus achieving said blockade, at least partial, of the reticuloendothelial system with cells.

39. The substance of claim 36, **characterized in that** said component comprises said cells, with which the reticuloendothelial system is blocked, at least partially, due to their rapid elimination from circulation in the bloodstream, sufficient enough to enable said blockade of the reticuloendothelial system.

40. The substance of claim 36, **characterized in that** said cells, which cause said blockade of the reticuloendothelial system, are erythrocytes.

41. The substance of claim 36, **characterized in that** said cells, which cause said blockade of the reticuloendothelial system, are thrombocytes.

42. The substance of claim 36, **characterized in that** said cells, which cause said blockade of the reticuloendothelial system, are leucocytes.

43. The substance of claim 36, **characterized in that** said cells are the cells administered to the organism by transfusion of donor's material or organism's material.

44. The substance of claim 36, **characterized in that** said cells are the processed variants of the organism cells or allogeneic cells.

45. The substance of claim 44, **characterized in that** said processed variants of the organism cells or allogeneic cells are the cells, inter alia, incubated with specific antibody that directly or indirectly recognize and bind to said variants of the organism's cells or allogeneic cells.

46. The substance of claim 45, **characterized in that** said specific antibody is an antibody (or analogs thereof), which are highly homologous to the antibodies of the organism, including autologous, allogeneic, humanized or chimeric antibody.

47. The substance of claim 45, **characterized in that** said specific antibody is a monoclonal antibody.

48. The substance of the claims 44-47, **characterized in that** said cells are a part of said component.

49. The substance of claim 36, **characterized in that** said cells are the cells that have not left the organism.

50. The substance of claim 36, **characterized in that** said component specifically interacts with the cells circulating in the bloodstream, and this interaction induces (leads to) their elimination from the bloodstream by the reticuloendothelial system, which results in said blockade of the reticuloendothelial system.

51. The substance of claim 50, **characterized in that** said interaction of said component with the cells circulating in the bloodstream is binding of said component to said cells.

52. The substance of claim 36, **characterized in that** said component noncovalently interacts with the cells circulating in the bloodstream.

53. The substance of claim 36, **characterized in that** said component forms a specific complex with said cells due to direct or indirect recognition and binding to said cells.

54. The substance of claim 53, **characterized in that** said component comprises at least an antibody, which forms said specific complex with said cells due to direct or indirect recognition and binding to said cells.

55. The substance of claim 54, **characterized in that** said antibody is targeted against erythrocytes of the organism.

56. The substance of claims 53, 54, **characterized in that** said direct or indirect recognition and binding of said component to the cells causes phagocytosis of said cells by other cells of the organism.

57. The substance of claim 53, 54, **characterized in that** said direct or indirect recognition and binding of said component to cells causes primarily the Fc-receptor-mediated phagocytosis of said cells by other cells of the organism.

58. The substance of claim 36, **characterized in that** said cells are agglutinated cells or are comprised in said component, or agglutinate under effect of said component.

59. The substance of claim 36, **characterized in that** said cells are damaged cells, and either are comprised in said component or are damaged under effect of said component.

60. The substance of claim 36, **characterized in that** said cells are modified cells, and either are comprised in said component, or are modified under effect of said component.

61. The substance of claim 60, **characterized in that** said modified cells are enzymatically modified, inter alia, by at least one of the following enzymes: neuraminidase, trypsin, galactose oxidase.

62. The substance of claim 60, **characterized in that** said modified cells are chemically modified, inter alia, by at least one of the following substances: glutaraldehyde, hydrogen peroxide.

63. The substance of claim 36, **characterized in that** said component comprises at least several antibodies against the organism's cells or allogeneic variants thereof, said antibodies are at least monoclonal or at least highly homologous to the antibodies of the organism (or their combinations and variants), including autologous, allogeneic, humanized or chimeric antibodies.

64. The substance of claim 36, **characterized in that** said component differs from an erythrocyte or a modified erythrocyte.

65. The substance of claim 36, administration of which leads to the blockade of the reticuloendothelial system, wherein the half-life of said agent's circulation in the bloodstream increases by at least 3 times as compared to the half-life of the agent's circulation without administration of the substance.

66. The substance of claim 36, **characterized in that** the dose of active components, the presence of which causes the blockade of the reticuloendothelial system, does not exceed 5 mg/kg of the organism's weight.

67. The substance of claim 36, administration of which enhances the efficiency of targeted delivery of said agent by at least 3 times as compared to that in the case of delivery of the agent to a target without administration of the substance.

68. The substance of claim 36, administration of which enhances the diagnostic or therapeutic efficiency of said agent by at least 2 times as compared to that in the case of delivery of the agent to a target without administration of the substance.

69. The substance of claim 36, **characterized in that** said agent comprises, inter alia, of at least one of the following nanoparticles or microparticles: magnetic, fluorescent, protein (including a cross-linked, polymerized or aggregate protein), polymer, including that consisting of at least one of the following polymers: polystyrene, dextran, polypeptide, polylactide glycolic acid, or block-copolymers) or crystalline (gold, silver, semiconductor, or metallic) nanoparticle or microparticle.

70. The substance of claim 36, **characterized in that** said agent is used for diagnostics or therapy of diseases or conditions of the organism that include one or more diseases from the following list: cancer, atherosclerosis, stroke, myocardial infarction, including diagnostics of diseases or conditions of the organism using angio contrast examination.

71. The substance of claim 36, **characterized in that** said agent performs a visualizing function due to generation of a detectible signal, including at least one of the following signals: fluorescent, luminescent, PET-signal, MRI-contrasting signal, X-ray contrasting signal, magnetic signal, or plasmon resonance signal, or a signal due to absorption of light or other electromagnetic or acoustic waves.

72. The substance of claim 36, **characterized in that** said agent implements a therapeutic function, inter alia, by means of delivery to targets of cytostatic or cytotoxic compounds, or medicinal compounds, including low molecular weight compounds, enzyme, radioactive, chemotherapeutic compounds, compounds for photodynamic therapy, or hyperthermia.

73. A method for diagnostics or therapy of diseases or conditions of the organism, wherein:
i) a substance according to any one of Claims 1-72 is administered to the organism;
ii) an agent that performs a diagnostic or therapeutic action is administered to the organism before or after the said substance, or co-administered with said substance.

74. The method of claim 73, **characterized in that** said agent is administered not less than 6 hours and not more than 18 hours after administration of the substance.

75. The method of claim 73, **characterized in that** said administration of said substance to the organism enhances the efficiency of diagnostic or therapeutic action of said agent by prolonging the blood circulation time of said agent.

76. The method of claim 73, **characterized in that** along with administration of said substance and said agent, a recovery preparation is administered to the organism, wherein said agent increases (as compared to the cases without administration of the recovery preparation) the level of cells or molecules, clearance of which from the bloodstream is caused by said component of said substance.

77. The method of claim 76, **characterized in that** said recovery preparation comprises the blood of the organism itself or said blood constituents (components), or donor's blood, or donor's blood constituents (components).

78. A substance for delivery of agent to the organism, which comprises at least a component that, upon administration of said substance into organism, stimulates elimination from the bloodstream by the reticuloendothelial system of at least the objects, which circulate in the bloodstream but do not represent artificially created nanoparticles or microparticles, or opsonins, which non-specifically bind to said component, and said elimination of said objects causes blockade, at least partial, of the reticuloendothelial system.

79. The substance of claim 78, **characterized in that** the mass of said component does not exceed 20 % of the mass of the objects that cause the blockade of the reticuloendothelial system.

80. A substance for delivery of the agent to the organism, comprising at least a component, which, upon administration to the organism, causes blockade, at least partial, of the reticuloendothelial system with cells.

81. A method for delivery of the agent to the organism, wherein:
i) a substance according to any one of Claims 78-80 is administered to the organism;
ii) said agent, which is not comprised in said substance is administered to the organism before or after said substance or co-administered with said substance.

82. A substance administered to the organism in order to increase the circulation time of an agent in the bloodstream, said substance comprising at least:
(i) component 1, which, when administered to the organism, intensifies elimination from circulation in the bloodstream of erythrocytes or thrombocytes, which have been naturally available in the organism or got to the organism artificially, as compared to their elimination rate without administration of said component,
(or)
(ii) component 2 that comprises at least an antibody (or variants thereof), which is highly homologous to the antibodies of the organism, including autologous, allogeneic, humanized or chimeric antibody, capable of forming, directly or indirectly, a specific complex with the objects, which have been naturally available in the organism or got in the organism artificially.

83. The substance of claim 82, **characterized in that** said antibody of said component 2 is gamma-immunoglobulin.

84. The substance of claim 82, **characterized in that** said erythrocytes or thrombocytes that got to the bloodstream artificially, or said objects that have been got to the organism artificially, have been administered to the organism by transfusion of the donor's material or material of the organism.

85. The substance of claim 82, **characterized in that** said erythrocytes or thrombocytes that got to the bloodstream artificially, or said objects that have been got to the organism artificially, constitute a component of said substance.

86. The substance of claim 82, **characterized in that** said erythrocytes or thrombocytes that that got to the bloodstream artificially, constitute a part of said component 1 of the substance.

87. The substance of claim 82, **characterized in that** said erythrocytes or thrombocytes, which are eliminated at the increased rate from circulation in the bloodstream, are processed variants of the organism's cells or of allogeneic cells, or are one of the components of the substance, or got in the organism artificially.

88. The substance of claim 87, **characterized in that** said processing of variants of the organism's cells or of allogeneic cells comprises incubation with specific antibody that directly or indirectly recognize and bind to said variants of the organism's cells or allogeneic cells.

89. The substance of claim 88, **characterized in that** said specific antibody are monoclonal antibody.

90. The substance of claim 82, **characterized in that** said objects are erythrocytes.

91. The substance of claim 82, **characterized in that** said objects are leucocytes.

92. The substance of claim 82, **characterized in that** said objects are thrombocytes.

93. The substance of claim 82, **characterized in that** said objects are albumins.

94. The substance of claim 82, **characterized in that** said objects are immunoglobulins.

95. The substance of claim 82, **characterized in that** said component 1 or said component 2 specifically interacts with said erythrocytes, or thrombocytes, or objects, and this interaction causes enhanced elimination from circulation of said erythrocytes, or thrombocytes, or objects.

96. The substance of claim 95, **characterized in that** said interaction of said component 1 or said component 2 with said erythrocytes, or thrombocytes, or objects is binding of the component to said erythrocytes, or thrombocytes, or objects.

97. The substance of claim 82, **characterized in that** said component 1 or said component 2 noncovalently interacts with erythrocytes, or thrombocytes, or objects circulating in the bloodstream.

98. The substance of claim 82, **characterized in that** said agent is nanoparticles or microparticles (including the particles comprising nanoparticles or micro-particles).

99. The substance of claim 82, **characterized in that** said increased elimination of erythrocytes or thrombocytes is caused by their ageing under effect of said component 1.

100. The substance of claim 82, **characterized in that** said increased elimination of erythrocytes, or thrombocytes, or objects is caused by their agglutination under effect of said component 1 or said component 2 of the substance.

101. The substance of claim 82, **characterized in that** said increased elimination of erythrocytes or thrombocytes is caused by their damage under effect of said component 1.

102. The substance of claim 82, **characterized in that** said component 1 or said component 2 causes increased phagocytizing activity of the organism's cells, which induces (leads to) the increased elimination from circulation of erythrocytes, or thrombocytes, or objects.

103. The substance of claim 82, **characterized in that** said increase of the circulation time of said agent is caused by at least partial blockade of the reticuloendothelial system of the organism due to elimination from circulation in the bloodstream of said erythrocytes, or thrombocytes, or objects.

104. The substance of claim 82, **characterized in that** said component 1 forms a specific complex with erythrocytes or thrombocytes due to direct or indirect recognition and binding to said erythrocytes or thrombocytes.

105. The substance of claim 82, **characterized in that** said antibody of said component 2 forms said specific complex with said objects due to direct or indirect recognition and binding to said objects.

106. The substance of claim 105, **characterized in that** said antibodies are targeted against erythrocytes of the organism or allogeneic analogs thereof.

107. The substance of claim 105, **characterized in that** said antibodies are targeted against thrombocytes of the organism or allogeneic analogs thereof.

108. The substance of claim 105, **characterized in that** said antibodies are targeted against leucocytes of the organism or allogeneic analogs thereof.

109. The substance of claims 104, 105, **characterized in that** said direct or indirect recognition and binding of said component 1 or said component 2 to erythrocytes, or to thrombocytes, or to objects causes primarily phagocytosis of erythrocytes, or thrombocytes, or objects mediated by Fc-receptor.

110. The substance of claim 82, **characterized in that** said component 1 or said component 2 is a chemical compound, molecule, particle or cell (including bacterium, leukocyte, etc.) or a combination of these objects but does not comprise erythrocytes.

111. The substance of claim 82, **characterized in that** said component 1 or said component 2 is a chemical compound, molecule, particle or cell (including bacterium, leukocyte, etc.) or a combination of these objects but does not comprise any objects obtained through any modification of erythrocytes.

112. The substance of claim 82, **characterized in that** said antibody of said component 2 are monoclonal antibodies, or said component 1 comprises at least a monoclonal antibody.

113. The substance of claim 82, **characterized in that** said component 1, or said component 2 comprises at least a complex of molecules or cells (or allogeneic variants thereof), extracted from the organism, and antibodies directly or indirectly bound therewith.

114. The substance of claim 82, **characterized in that** said component 1 or said component 2 comprises at least several antibodies (or combinations and variants thereof), which are at least monoclonal or at least highly homologous to the antibodies of the organism (or their combinations and variants), including autologous, allogeneic, humanized or chimeric antibodies, against the organism's cells or allogeneic variants thereof.

115. The substance of claim 82, administration of which to the organism results in said increase of the circulation time of said agent in the bloodstream so that the half-life of the agent's circulation in the bloodstream increases by at least 3 times as compared to the half-life of the agent's circulation without administration of the substance.

116. The substance of claim 82, **characterized in that** said agent is used for diagnostics, or monitoring, or therapy of a disease, wherein the increase of the circulation time of said agent due to administration of the substance enhances the efficiency of diagnostics or therapy of the disease.

117. A method for increasing the circulation time of an agent in the bloodstream, wherein:
i) the substance according to claims 82-116 is administered to the organism;
ii) said agent is administered to the organism.

118. The method of claim 117, **characterized in that** the administration of the agent is used for diagnostics, or monitoring, or therapy, wherein the increase of the circulation time of the agent in the bloodstream of the organism enhances efficiency of the diagnostics or therapy.

119. The method of claim 117, **characterized in that** the dose of the substance for administering thereof to the organism is selected to increase the circulation time by a predetermined multiplier for optimization of the balance between the beneficial effect of the increased circulation time and possible negative effects.

120. The method of claim 117, **characterized in that** administration of said substance or variants thereof is repeated several times.

121. The method of claim 117, **characterized in that** along with administration of said agent and said substance to the organism, a recovery preparation is administered, said recovery preparation causing an increase of level of cells or molecules, which are cleared (eliminated) from circulation in the bloodstream at an increased rate when affected said substance, as compared to that level without administration of the recovery preparation.

122. The method of claim 121, **characterized in that** said recovery preparation comprises the blood of the organism itself or the blood constituents (components), or donor's blood, or donor's blood constituents (components).

123. A method for promoting products to the market, comprising at least promotion for research, diagnostics, monitoring or therapy of diseases or conditions of the organism, of at least
(i) the substance of claims 1-72,
or (ii) the method of claims 73-77,
or (ii) the substance of claims 78-80,
or (ii) the method of claim 81,
or (ii) the substance of claims 82-116,
or (ii) the method of claims 117-122.

124. The method of claim 123, **characterized in that** the promotion is carried out by using a leaflet inserted into the package with a commercial substance, comprising at least the substance of claims 1-72, or the substance of claims 78-80, or the substance of claims 82-116.

125. A business method, comprising at least marketing for diagnostics and therapy, or enhancement of the efficiency of diagnostics or therapy, or for delivery of agent to the organism, or for increase of the circulation time of the agent in the bloodstream of:
(i) the substance of claims 1-72,
or (ii) the method of claims 73-77,
or (ii) the substance of claims 78-80,
or (ii) the method of claim 81,
or (ii) the substance of claims 82-116,
or (ii) the method of claims 117-122.
